Europäisches Patentamt

European Patent Office

Office européen des brevets

(19)

(11) Numéro de publication: **0 075 524**
**B1**

(12) # FASCICULE DE BREVET EUROPEEN

(45) Date de publication du fascicule du brevet:
**12.09.84**

(51) Int. Cl.³: **C 07 C 69/52**, C 07 C 67/38

(21) Numéro de dépôt: **82420097.6**

(22) Date de dépôt: **09.07.82**

(54) **Procédé de carbonylation des diènes conjugués en présence d'un catalyseur au palladium avec recyclage dudit catalyseur.**

(30) Priorité: **16.09.81 FR 8117464**

(43) Date de publication de la demande:
**30.03.83 Bulletin 83/13**

(45) Mention de la délivrance du brevet:
**12.09.84 Bulletin 84/37**

(84) Etats contractants désignés:
**AT BE CH DE FR GB IT LI LU NL SE**

(56) Documents cités:
**GB - A - 2 014 136**

(73) Titulaire: **RHONE-POULENC CHIMIE DE BASE, 25, quai Paul Doumer, F-92408 Courbevoie (FR)**

(72) Inventeur: **Jenck, Jean, 11, rue Lakanal, F-69100 Villeurbanne (FR)**

(74) Mandataire: **Varniere-Grange, Monique et al, RHONE-POULENC RECHERCHES Service Brevets Chimie et Polymères Centre de Recherches de Saint-Fons B.P. 62, F-69192 St-Fons Cédex (FR)**

## Description

La présente invention a pour objet un procédé de préparation d'esters d'acides carboxyliques β,γ-insaturés par carbonylation de diènes conjugués par de l'oxyde de carbone en présence d'un alcool, d'un hydracide halogéné et d'un catalyseur au palladium avec récupération et recyclage dudit catalyseur.

Il est connu d'après le brevet japonais No 48.5564 de préparer des monoesters d'acides carboxyliques β,γ-insaturés par carbonylation des diènes conjugués par de l'oxyde de carbone, en présence d'un monoalcool, d'un catalyseur au palladium non halogéné et d'un hydracide halogéné, à une température de l'ordre de 100° C et une pression d'oxyde de carbone de l'ordre de 100 bar.

Aucune information quant à une méthode de récupération du catalyseur n'est indiquée.

La titulaire a montré dans sa demande de brevet français No 81.01205 déposée le 23 janvier 1981, que la sélectivité en esters recherchés, le taux de conversion des diènes conjugués mis en œuvre et la stabilité du catalyseur au palladium sont améliorés si le milieu soumis à la carbonylation contient en outre un sel d'onium quaternaire d'un élément du groupe VB choisi parmi l'azote, le phosphore et l'arsenic et présentant un anion choisi parmi les bases «dures» ou «intermédiaires» (selon la définition donnée par R. Pearson dans «J. Chem. Ed.», 45, 581-7, 1968).

Cette demande n'indique pas non plus comment récupérer le catalyseur au palladium en vue de son recyclage.

La titulaire a maintenant trouvé un procédé de préparation d'esters d'acides carboxyliques β,γ-insaturés par carbonylation des diènes conjugués pouvant être utilisé en continu aussi bien qu'en discontinu.

La présente invention a pour objet un procédé de préparation d'esters carboxyliques β,γ-insaturés par carbonylation de diènes conjugués par de l'oxyde de carbone, en présence d'un alcool, d'un hydracide halogéné et d'un catalyseur au palladium, ledit procédé étant caractérisé en ce que:
— les produits issus de l'opération de carbonylation sont soumis, en présence d'un chlorure ou d'un bromure d'onium quaternaire choisi parmi les chlorures ou bromures d'ammonium, de phosphonium et d'arsonium quaternaires présentant une température de fusion inférieure à celle à laquelle est réalisée l'opération de carbonylation, à une opération de distillation dans des conditions de température et de pression permettant de récupérer:

une phase gazeuse contenant l'ester d'acide carboxylique β,γ-insaturé correspondant au diène et à l'alcool mis en œuvre, le diène et l'alcool non réagis ainsi que les produits légers volatils, et

une phase liquide homogène constituée d'un mélange du chlorure ou bromure d'onium quaternaire et du catalyseur au palladium ainsi que des produits lourds non volatils;
— après élimination éventuelle des produits lourds, ledit mélange de chlorure ou bromure d'onium quaternaire et de catalyseur au palladium est recyclé sous la forme d'un liquide homogène dans le milieu soumis à l'opération de carbonylation.

Les opérations de carbonylation des diènes conjugués en présence d'un catalyseur au palladium peuvent généralement être réalisées à une température comprise entre environ 50 et 160° C, de préférence entre 80 et 150° C, la pression d'oxyde de carbone étant, de préférence, comprise entre 50 et 250 bar.

Parmi les chlorures ou bromures d'onium quaternaire pouvant être présents lors de l'opération de distillation, on peut citer: le chlorure de tétrabutylphosphonium fondant vers 65° C; les chlorures de méthyl tri(-octyl, -nonyl, ou -décyl)ammonium ou leurs mélanges commercialisés sous le nom d'Aliquat 336 ou d'Adogen 464 et fondant vers 100° C; le chlorure de méthyltributylammonium fondant vers 102° C; le chlorure de benzylbutyldiméthylammonium fondant vers 62° C; le chlorure de benzylhexadécyldiméthylammonium fondant vers 60° C; le chlorure de benzyldiméthyltétradécylammonium fondant vers 62° C; le bromure d'hexadécyltributylphos-phonium fondant vers 57° C; le bromure de tétrabutylammonium fondant vers 102° C; le bromure de tétradodécylammonium fondant vers 91° C; le bromure de tétraheptylammonium fondant vers 87° C; le bromure de tétrahexylammonium fondant vers 90° C; le bromure de tétradécylammonium fondant vers 88° C; le bromure de tétraoctadécylammonium fondant vers 103° C; le bromure de tétraoctylammonium fondant vers 94° C; le bromure de tétrapentylammonium fondant vers 100° C; le bromure de tributylheptylammonium fondant vers 58° C; le bromure de tributylpentylammonium fondant vers 64° C.

La quantité de chlorure ou bromure d'onium quaternaire pouvant être présente lors de l'opération de distillation varie dans de très larges limites, la quantité minimum correspondant à un rapport molaire cation onium quaternaire/palladium de 2 environ. Economiquement, il est intéressant d'utiliser une quantité de chlorure ou bromure d'onium quaternaire correspondant à un rapport molaire compris entre environ 5 et 250, bien que des quantités supérieures ne soient aucunement nocives au bon déroulement des opérations de distillation et de carbonylation.

Le procédé faisant l'objet de la présente invention est particulièrement bien adapté à la carbonylation de diènes conjugués présentant dans leur molécule le squelette butadiène-1,3 tels que:

les diènes aliphatiques linéaires ou ramifiés, contenant de 4 à 12 atomes de carbone, et de préférence de 4 à 8 atomes de carbone, éventuellement substitués par des groupes inertes tels que: phényle, cyclohexyle, nitro, oxo et alcoxycarbo-nyle;

les diènes cycliques contenant de 6 à 8 atomes de carbone.

Comme exemples concrets de diène conjugué, on peut citer le butadiène-1,3, l'isoprène, le pipérylène, l'hexadiène-1,3, l'hexadiène-2,4, le chloroprène, le cyclohexyl-1 butadiène-1,3, le phényl-1 butadiène-1,3, l'octadiène-2,4, le méthyl-3 pentadiène-1,3, le méthyl-2 pentadiène-2,4, le cyclohexadiène-1,3, le cyclooctadiène-1,3, etc.

L'alcool pouvant être mis en œuvre pour réaliser l'opération de carbonylation est un monoalcool linéaire ou ramifié contenant de 1 à 4 atomes de carbone, de préférence de 1 à 3 atomes de carbone.

L'hydracide halogéné mis en œuvre est l'acide chlorhydrique ou l'acide bromhydrique qui peut être introduit dans le milieu de carbonylation sous forme gazeuse ou sous la forme d'un composé organique susceptible de libérer de l'HCl ou de l'HBr dans le milieu, par exemple sous forme de chloro-1 butène-2, de chloro-3 butène-1, de bromo-1 butène-2 ou de bromo-3 butène-1, dans le cas de la carbonylation du butadiène. L'HCl et les composés organiques susceptibles de libérer de l'HCl sont préférés.

Parmi les catalyseurs au palladium présents lors de l'opération de carbonylation, on peut citer:

le palladium métallique déposé sur un support, tel que le charbon, l'alumine, la silice, etc.,

les oxydes de palladium,

les sels ou complexes de palladium II dont l'anion coordiné au cation Pd est une base dure ou intermédiaire, notamment les sels ou les complexes $\pi$-allyliques du Pd dont l'anion coordiné au cation Pd est choisi parmi les anions suivants: carboxylates, tels que formiate, acétate, propionate, benzoate; $SO_4^{2-}$, $NO_3^-$; acétyl-acétonate; halogénures tels que $Cl^-$, et $Br^-$, et de préférence $Cl^-$;

ou les complexes de palladium zéro comprenant des ligands organiques ne contenant pas d'éléments du groupe VB, complexes tels que le bis(dibenzalacétone) Pd ou le bis(cyclooctadiène-1,5) Pd.

Les quantités de réactifs à utiliser pour réaliser l'opération de carbonylation peuvent varier entre des limites très larges; il est évident que lesdites quantités seront choisies de manière que le procédé soit économiquement intéressant.

Ainsi, bien qu'il soit possible d'utiliser de 0,5 à 10 fois la quantité d'alcool stœchiométriquement nécessaire, il est préférable, afin d'avoir une conversion maximum du diène conjugué tout en évitant de trop diluer le milieu par de l'alcool, de réaliser le procédé avec un rapport molaire alcool/diène conjugué compris entre environ 0,8 et 5 et de préférence voisin de 1.

De même la bonne activité des catalyseurs au palladium permet l'emploi desdits catalyseurs en quantité très faible (correspondant à un rapport molaire diène conjugué/palladium de l'ordre de 2500); l'emploi d'une quantité élevée de catalyseur (correspondant à un rapport molaire diène conjugué/palladium de l'ordre de 100) n'est pas nuisible; le but recherché étant de réaliser une opération de carbonylation suffisamment rapide et sélective, sans dépenser trop de catalyseur, un rapport diène conjugué/palladium compris entre environ 100 et 2000 est généralement préférable.

La quantité d'hydracide halogéné à utiliser correspond à un rapport molaire hydracide halogéné/palladium supérieur à 2. Toutefois, afin d'éviter tout risque de dégradation de l'alcool en halogénures d'alkyle et oxydes de dialkyle (dégradation due à une concentration trop importante du milieu en hydracide), un rapport molaire hydracide halogéné/palladium compris entre environ 2 et 100, et de préférence entre environ 5 et 60, pourra être favorablement choisi.

Le but de l'opération de distillation étant de séparer le catalyseur au palladium du produit principal de l'opération de carbonylation, à savoir de l'ester d'acide carboxylique $\beta,\gamma$-insaturé correspondant au diène et à l'alcool mis en œuvre, les conditions de température et de pression auxquelles est réalisée l'opération de distillation sont adaptées de manière à vaporiser ledit ester d'acide carboxylique $\beta,\gamma$-insaturé, le diène et l'alcool non réagis ainsi que les produits légers volatils et à récupérer le catalyseur au palladium et le chlorure ou bromure d'onium quaternaire dans le résidu liquide de distillation contenant les produits lourds non volatils.

On entend par «produits légers volatils» les produits organiques présentant un point d'ébullition voisin ou inférieur à celui de l'ester d'acide carboxylique $\beta,\gamma$-insaturé obtenu.

On entend par «produits lourds non volatils» ceux présentant un point d'ébullition supérieur à celui de l'ester d'acide carboxylique $\beta,\gamma$-insaturé obtenu.

Il est évident que la répartition entre «produits légers volatils» et «produits lourds non volatils» varie selon la nature du diène et de l'alcool de départ.

Ainsi, lorsque le diène et l'alcool choisis sont le butadiène et l'éthanol et l'hydracide halogéné HCl, constituent:

— la phase gazeuse, notamment les produits suivants: butadiène, butènes, chlorobutènes, éthoxybutènes, dimères du butadiène (essentiellement du vinyl-4 cyclohexène), penténoates d'éthyle, méthyl-2 butène-3 oate d'éthyle, éthanol, HCl et chlorure d'éthyle;

— le résidu liquide de distillation, notamment les produits suivants: dimère carbonylé (nonadiène-3,8 oate d'éthyle), diesters en $C_6$ (en majorité du méthyl-2 glutarate de diéthyle) et oligomères du butadiène.

Par contre, lorsque le diène et l'alcool choisis sont l'hexadiène-1,3 et l'éthanol, le résidu de distillation contient entre autres le dimère du diène car celui-ci est moins volatil que l'ester d'acide carboxylique $\beta,\gamma$-insaturé obtenu.

De même, certains sous-produits de la réaction de carbonylation peuvent ne pas se former; ainsi lorsque le diène choisi est l'isoprène, on ne constate pas de formation de dimère carbonylé.

La quantité de chlorure ou bromure d'onium quaternaire nécessaire au bon déroulement de l'opération de distillation peut être introduite dans le milieu issu de l'opération de carbonylation en totalité après la première opération de carbonylation ou par fractions au cours d'une série d'opérations de carbonylation-distillation. La présence de chlorures ou bromures d'onium quaternaire jouant un rôle favorable lors de la carbonylation, il est intéressant de mettre en œuvre le chlorure ou bromure d'onium quaternaire en tout ou partie pendant la carbonylation. En effet, il a été constaté (cela fait l'objet de la demande de brevet français N° 81.01205 déposée le 23 janvier 1981 au nom de la titulaire) que les diènes conjugués peuvent être carbonylés en présence d'un alcool, d'un hydracide halogéné, d'un catalyseur au palladium et d'un chlorure ou bromure d'onium quaternaire, notamment en présence des chlorures ou bromures d'ammonium, de phosphonium ou arsonium quaternaires mis en œuvre pour réaliser l'opération de distillation de la présente invention; un effet bénéfique, notamment sur le taux de conversion des diènes et sur la sélectivité en esters carboxyliques $\beta,\gamma$-insaturés recherchés, a été constaté pour un rapport molaire cation onium/palladium d'au moins 0,5 et tout particulièrement compris entre 1 et 15; des quantités beaucoup plus importantes de chlorure ou bromure d'onium peuvent toutefois être utilisées sans nocivité.

L'opération de recyclage dans le milieu soumis à l'opération de carbonylation du mélange de chlorure ou bromure d'onium quaternaire et de catalyseur au palladium sous la forme d'un liquide homogène peut être réalisée par simple recyclage directement dans le milieu à carbonyler de la phase liquide homogène séparée lors de l'opération de distillation et constituée dudit mélange de chlorure ou bromure d'onium quaternaire et de catalyseur au palladium et des produits lourds non volatils.

La titulaire a constaté qu'il est possible de réaliser plusieurs cycles successifs d'opérations de carbonylation, distillation et recyclage. La phase liquide homogène à recycler s'enrichit au fur et à mesure en produits lourds non volatils, ce qui augmente progressivement le volume de la phase liquide à recycler. Industriellement, il est préférable de ne pas avoir à recycler une trop grande quantité de liquide.

Un autre but de la présente invention est de pousser beaucoup plus loin les limites du procédé ci-dessus, limites dues à la présence de produits lourds dans la phase liquide homogène séparée par distillation, en faisant subir à ladite phase liquide homogène un traitement d'élimination totale ou partielle des produits lourds et en recyclant dans le milieu soumis à la carbonylation le mélange de chlorure ou bromure d'onium quaternaire et de catalyseur au palladium sous la forme d'un liquide homogène.

Ledit traitement d'élimination des produits lourds peut être réalisé sur tout ou partie de la phase liquide homogène récupérée (après distillation) soit après chaque cycle de carbonylation-distillation, soit périodiquement après une série de cycles de carbonylation-distillation-recyclage.

La présente invention a également pour objet les différents modes d'élimination des produits lourds et de recyclage du mélange de chlorure ou bromure d'onium quaternaire et de catalyseur au palladium sous la forme d'un liquide homogène.

Le premier mode est une méthode de séparation biphasique liquide/liquide, qui consiste:
— à mettre en contact la phase liquide homogène, constituée du mélange de chlorure ou bromure d'onium quaternaire et de catalyseur au palladium et des produits lourds non volatils, avec un solvant apolaire hydrocarboné aliphatique ou cycloaliphatique, un solvant polaire non miscible dans ledit solvant apolaire et éventuellement une quantité supplémentaire de chlorure ou bromure d'onium quaternaire;
— après décantation de la phase polaire et de la phase apolaire formées lors de l'opération de mise en contact, à séparer la phase apolaire contenant les produits lourds à récupérer, la phase polaire contenant le mélange de chlorure ou bromure d'onium quaternaire et de catalyseur au palladium et à recycler vers le milieu soumis à l'opération de carbonylation le mélange de chlorure ou bromure d'onium quaternaire et de catalyseur au palladium soit sous la forme d'une solution alcoolique, soit à l'état fondu après élimination du solvant polaire; l'opération de mise en contact étant réalisée avec:
— une quantité de chlorure ou bromure d'onium quaternaire correspondant à un rapport molaire cation onium quaternaire/palladium d'au moins 20 environ;
— des quantités de solvant polaire et de solvant hydrocarboné apolaire au moins égales à celles nécessaires à la décantation du milieu de mise en contact en une phase polaire et une phase apolaire.

On entend par «phase polaire» la phase dont le solvant est constitué par le solvant polaire présent lors de l'opération de mise en contact.

On entend par «solvant polaire non miscible» avec le solvant apolaire les solvants de constante diélectrique supérieure à 20 environ (valeur mesurée à 20-25° C) et de préférence supérieure à 30 environ, présentant une séparation de phase avec ledit solvant apolaire, ainsi que les mono-alcools aliphatiques linéaires ou ramifiés contenant de 1 à 4 atomes de carbone, et de préférence de 1 à 3 atomes de carbone.

A titre d'exemple de solvant polaire pouvant être mis en œuvre on peut citer: l'eau ($\varepsilon=78,4$); l'acétonitrile ($\varepsilon=37,5$); le N,N-diméthylformamide ($\varepsilon=36,7$); le diméthylsulfoxyde ($\varepsilon=48$); le nitrométhane ($\varepsilon=35,9$); la N-méthyl-pyrrolidone ($\varepsilon=31$) ainsi que le méthanol, l'éthanol, le propanol, ou l'isopropanol.

Pour une raison de simplicité de réalisation de l'ensemble du procédé faisant l'objet de la présente invention, on utilisera de préférence comme solvant polaire, pour réaliser ladite opération de mise en contact, l'alcool mis en œuvre pour réaliser l'opération de carbonylation; la solution alcoolique de chlorure ou bromure d'onium quaternaire et de catalyseur au palladium ainsi obtenue peut être directement recyclée dans le milieu soumis à l'opération de carbonylation.

Toutefois, il peut être intéressant, pour une meilleure séparation des deux phases, de réaliser l'opération de mise en contact à l'aide d'un alcool différent de celui utilisé pour l'opération de carbonylation, d'eau ou d'un des solvants polaires non alcooliques ci-dessus cités; il est alors nécessaire d'éliminer par évaporation le solvant de la phase polaire récupérée et éventuellement de dissoudre le mélange de chlorure ou bromure d'onium quaternaire et de catalyseur au palladium dans l'alcool utilisé pour réaliser l'opération suivante de carbonylation.

On entend par «phase apolaire» la phase dont le solvant est constitué par le solvant hydrocarboné aliphatique ou cycloaliphatique apolaire présent lors de l'opération de mise en contact.

On entend par solvant hydrocarboné aliphatique ou cycloaliphatique apolaire les hydrocarbures aliphatiques ou cycloaliphatiques présentant une constante diélectrique inférieure à 2,3 (valeur mesurée à 20-25°C) et tout particulièrement inférieure à 2,1 (à 20-25°C). Parmi ceux-ci on peut citer: le pentane ($\varepsilon$=1,84), l'isopentane ($\varepsilon$=1,84), l'hexane ($\varepsilon$=1,89), le cyclohexane ($\varepsilon$=2,02), l'octane ($\varepsilon$=1,95), l'isooctane ($\varepsilon$=1,94), le cyclooctane ($\varepsilon$=1,95), le décane ($\varepsilon$=1,99), le dodécane ($\varepsilon$=2,01), le tétradécane, l'hexadécane ou leurs mélanges du type éther de pétrole.

La séparation entre la phase polaire et la phase apolaire est d'autant mieux réalisée que:
— le rapport molaire cation onium quaternaire/palladium est élevé;
— le rapport pondéral chlorure ou bromure d'onium quaternaire/solvant polaire est élevé;
— la différence entre la constante diélectrique du solvant polaire et celle du solvant hydrocarboné apolaire est grande.

Il est évident qu'économiquement il n'est pas intéressant d'utiliser du chlorure ou bromure d'onium quaternaire en trop grand excès, ni de mettre en œuvre de trop grandes quantités de solvant polaire.

Généralement, l'opération de mise en contact pourra être favorablement réalisée en ayant en présence:
— une quantité de chlorure ou bromure d'onium quaternaire correspondant à un rapport molaire cation onium/palladium compris entre environ 20 et 300 et de préférence entre 30 et 200;
— une quantité de solvant polaire correspondant à un rapport pondéral chlorure ou bromure d'onium quaternaire/solvant polaire d'au moins 0,1 environ et de préférence compris entre 0,25 et 30, et tout particulièrement entre 0,3 et 20;
— une quantité de solvant hydrocarboné apolaire correspondant à un rapport pondéral solvant hydrocarboné apolaire/solvant polaire supérieur à 1, de préférence supérieur à 2 environ, et tout particulièrement supérieur à 3.

Si désiré, une élimination plus poussée des produits lourds peut être obtenue en faisant subir à la phase polaire obtenue après décantation un traitement complémentaire consistant à soumettre ladite phase polaire à une ou plusieurs opérations d'extraction et de séparation à l'aide du solvant hydrocarboné apolaire.

L'opération de mise en contact est réalisée dans des conditions de température permettant d'obtenir une bonne séparation des deux phases et ce d'une manière économiquement intéressante. La miscibilité relative entre un solvant polaire et un solvant apolaire diminue généralement lorsque la température diminue; il a été constaté que la solubilité du chlorure ou bromure d'onium quaternaire dans le solvant polaire diminue également mais que celle-ci reste toutefois élevée; ainsi l'opération de mise en contact peut être réalisée à basse température et de préférence à une température voisine de la température ambiante.

Le deuxième mode d'élimination des produits lourds et de recyclage du mélange de chlorure ou bromure d'onium quaternaire et de catalyseur au palladium est une méthode de séparation solide-liquide, qui consiste:
— à mettre en contact les constituants de la phase liquide homogène séparée lors de l'opération de distillation avec un solvant apolaire hydrocarboné aliphatique ou cycloaliphatique, à une température inférieure ou égale à la température de prise en masse de la phase liquide homogène;
— à soumettre le système solide-liquide obtenu à une opération de filtration;
— à éliminer la solution de produits lourds non volatils dans ledit solvant apolaire;
— à transformer le résidu de filtration constitué d'un mélange de chlorure ou bromure d'onium quaternaire et de catalyseur au palladium en un liquide homogène par fusion ou par mise en solution dans un alcool, de préférence dans l'alcool mis en œuvre lors de l'opération de carbonylation, et
— à recycler ledit liquide homogène obtenu dans le milieu soumis à l'opération de carbonylation.

Ladite opération de mise en contact selon ce deuxième mode peut être réalisée:
— de préférence en refroidissant la phase liquide homogène séparée par distillation jusqu'à cristallisation du mélange de chlorure ou bromure d'onium quaternaire et de catalyseur au palladium et en additionnant au milieu obtenu le solvant apolaire en quantité au moins suffisante pour dissoudre les produits lourds non volatils;
— en additionnant à la phase liquide homogène séparée par distillation le solvant apolaire en quantité au moins suffisante pour dissoudre les produits lourds non volatils et en refroidissant le milieu obtenu jusqu'à cristallisation du mélange de chlorure ou bromure d'onium quaternaire et de catalyseur au palladium.

Les solvants hydrocarbonés aliphatiques ou cycloaliphatiques apolaires mis en œuvre sont ceux déjà mentionnés ci-dessus pour réaliser l'opération de mise en contact selon le premier mode d'élimination des produits lourds (à savoir selon la méthode de séparation biphasique liquide-liquide).

Ce deuxième mode d'élimination des produits lourds et de recyclage du mélange de chlorure ou bromure d'onium quaternaire et de catalyseur au palladium s'adapte particulièrement bien au traitement de résidus liquides homogènes de distillation ne contenant qu'une quantité limitée de produits lourds non volatils, par exemple pas plus de 10% en volume, lorsque le diène choisi est le butadiène et lorsque le sel d'onium quaternaire mis en œuvre est le chlorure de tétrabutylphosphonium.

Les entités élémentaires correspondant aux termes «moles» sont les suivantes:
— alcool: molécule-gramme
— diène conjugué: molécule-gramme
— hydracide halogéné: molécule-gramme
— palladium: atome-gramme
— cation onium quaternaire: ion-gramme

Les exemples suivants sont donnés à titre indicatif et ne peuvent être considérés comme une limite du domaine et de l'esprit de l'invention.

Les abréviations figurant dans les tableaux correspondant à ces exemples ont le sens suivant:
Ex: exemple
Bd: butadiène
ROH: alcool; Me: méthyl; Et: éthyl; Bu: n-butyl; Dod: dodécyl;
Pd(DBA)$_2$: bis(dibenzalacétone) Pd;
[$\langle$(Pd-Cl]$_2$: bis($\pi$-allyl palladium chlorure)
catalyseur: catalyseur au palladium
cocatalyseur: HCl ou produit de la réaction HCl + diène conjugué mis en œuvre;
onium: sel d'onium quaternaire
polaire: solvant polaire
alcane: solvant hydrocarboné apolaire
AN: acétonitrile
DMF: N,N-diméthylformamide
MR: masse réactionnelle issue de la carbonylation (contenant du butadiène non transformé ainsi que des produits volatils qui s'évaporent lors de l'opération ultérieure de mise en contact réalisée à température ambiante);
P$_3$: ester pentène-3 oïque
P$_4$: ester pentène-4 oïque
P$_2$: ester pentène-2 oïque
P': ester méthyl-2 butène-3 oïque
C$_9$: ester nonadiène-3,8 oïque
C$_6$: diesters alcoyliques en C$_6$ (en majorité du méthyl-2 glutarate de dialkyle)
HC$_8$: dimères de butadiène (essentiellement du vinyl-4 cyclohexène)
PA: ester pentanoïque et ester méthyl-2 butanoïque
ROC$_4$: alcoxy-3 butène-1 et alcoxy-1 butène-2
ClC$_4$: chloro-3 butène-1 et chloro-2 butène-2
ClPA: ester chloropentanoïque
Cl: chlorure d'alkyle oxyde de dialkyle (réaction parasite de HCl sur l'alcool)
TT: taux de conversion global du butadiène (en moles %)
RR: taux de conversion partiel (en moles %) pour chaque produit obtenu par rapport au butadiène chargé, avec:

$$TT = \sum_i RR_i$$

Ne sont pris en compte pour le calcul de TT que les RR des produits suivants: P$_3$, P$_4$, P$_2$, P', C$_9$, C$_6$, HC$_8$, PA, ROC$_4$, et ClPA; en effet, les chlorobutènes (ClC$_4$) sont équivalents à un mélange butadiène+HCl carbonylable en P$_3$.

RT: sélectivité (en moles %) pour chaque produit avec

$$RT = \frac{RR}{TT}$$

RRCl: taux de conversion partiel (en moles %) en chlorure d'éthyle et éther diéthylique, par rapport à l'éthanol chargé.

RRBr: taux de conversion partielle (en moles %) en bromure d'éthyle et éther diéthylique.

A: activité spécifique du catalyseur, exprimée en nombre de moles de P$_3$ obtenues par atg de Pd et par heure, plus de 95% des esters penténoïques linéaires obtenus étant constitués de P$_3$.

CO: CO gazeux technique contenant environ 0,8% en volume d'hydrogène qui n'intervient pas notablement pour la réaction de carbonylation.

■

*Exemple 1*

1a) *Etape de carbonylation*

Dans un autoclave de 125 cm³ en alliage nickel-molybdène de marque Hastelloy B$_2$, on introduit sous courant d'argon:
— 0,0774 g (soit 0,2115 mmole) de bis($\pi$-allyl-palladium chlorure) anhydre (0,423 matg de Pd);
— 12,5 g (soit 42,2 mmoles) de chlorure de tétrabutyl phosphonium ce qui correspond à un rapport molaire PBu$_4$$^+$Cl$^-$/Pd de 100;
— 12 g (soit 261 mmoles) d'éthanol;
— 0,19 g (soit 2,12 mmoles) de chloro-1 butène-2, ce qui correspond à un rapport molaire HCl/Pd de 5.

L'autoclave est fermé; on y transverse 14,5 g (soit 268,5 mmoles) de butadiène, ce qui correspond à un rapport molaire butadiène/Pd de 635 et à un rapport molaire alcool/butadiène de 1.

L'autoclave agité par secousses est porté à 120°C avec alimentation de CO à pression totale constante de 196 bars.

On laisse la réaction se dérouler pendant 2 heures à cette température. L'autoclave est ensuite refroidi à 15°C et on dégaze lentement.

Les conditions réactionnelles sont résumées dans le tableau IA.

On récupère 41,17 g d'une solution homogène jaune-verte contenant 5,2% en poids d'éthanol et 61,2% en poids de penténoates d'éthyle.

On donne au tableau IIA les résultats de l'analyse chromatographique en phase gazeuse de la solution obtenue ainsi que les RT et RR correspondant à chaque produit formé.

Le taux de conversion global du butadiène TT est de 80,7 moles %.

L'activité spécifique A du catalyseur est de 232 h$^{-1}$.

La solution homogène jaune-verte résultant de cette étape 1a) est séparée en fractions, une fraction de 20 g à soumettre à une opération de distillation 1b), et une fraction de 15,1 g à soumettre à une opération de distillation 1d).

1b) *Etape de distillation*

(Les conditions et les résultats de distillation sont résumés au tableau IIIA.)

On prend 20 g de la solution jaune obtenue, contenant 6,05 g de chlorure de tétrabutylphosphonium, sur laquelle on effectue une opération de distillation à une température de 84° C et sous une pression absolue de 3500 Pa. On obtient une quantité de condensat de distillation de 10,80 g exempt de Pd et contenant: 10,3 g de pentène-3 oate d'éthyle et un résidu de distillation de 7,5 g qui est un liquide homogène, limpide de couleur orangée. Ce résidu liquide à la température de distillation contient la totalité du catalyseur au palladium et du chlorure de tétrabutylphosphonium mis en œuvre soit:
— 21,9 mg (0,206 matg) de Pd
— 6,05 g (20,51 mmoles) de chlorure de phosphonium.

### 1c) *Recyclage dans l'autoclave de carbonylation*

On réalise une étape de carbonylation dans les conditions décrites à l'étape 1a) à partir de:
— 7,5 g de résidu liquide de distillation à l'étape 1b);
— 0,0933 g (soit 1,03 mmoles) de chloro-1 butène-2, ce qui correspond à un rapport molaire HCl/Pd de 5;
— 6 g (130,4 mmoles) d'éthanol;
— 6 g (111,2 mmoles) de butadiène, ce qui correspond à des rapports molaires butadiène/Pd=540 et alcool/butadiène=1,17.

Après 2 h de carbonylation à 120° C sous 196 bars, on récupère 19,02 g d'une solution verte contenant 4,6% en poids d'éthanol et 61% en poids de penténoates d'éthyle.

On donne au tableau IIA les résultats de l'analyse chromatographique en phase gazeuse de la solution obtenue ainsi que les RT et RR correspondant à chaque produit formé.

Le taux de conversion global du butadiène TT est de 86,5 moles %.

L'activité spécifique A du catalyseur est de 222 h$^-$.

On constate qu'il n'y a eu aucune perte de catalyseur et que l'activité de celui-ci a été conservée.

### 1d) *Etape de distillation*

On prend 15,1 g de la solution jaune-verte obtenue à l'étape de carbonylation 1a), contenant 4,55 g de chlorure de tétrabutyl phosphonium, sur laquelle on effectue une opération de distillation à une température de 84° C et sous une pression de 3100 Pa. On obtient une quantité de condensat de distillation de 8,4 g et un résidu de distillation liquide de couleur orangée de 4,95 g contenant:
— 16,5 mg (soit 0,155 matg) de Pd
— 4,55 g (soit 15,4 mmoles) de chlorure de phosphonium.

### 1e) *Extraction des produits lourds*

(Les conditions et les résultats de l'extraction sont résumés au tableau IV.)

On ajoute au résidu de distillation de l'étape 1d) 40 g de n-hexane et 13 g de méthanol.

La composition ainsi obtenue contient:
— 22,4% en poids de méthanol ce qui correspond à un rapport pondéral solvant apolaire/alcool de 3,1;
— 7,9% en poids de PBu$_4$$^+$Cl$^-$, ce qui correspond au rapport molaire PBu$_4$$^+$Cl$^-$/Pd de 100 et à un rapport pondéral PBu$_4$$^+$Cl$^-$/alcool de 0,35.

Le milieu se sépare en deux phases:
— une phase supérieure incolore (41,1 g) contenant l'hexane, le nonadiène-3,8 oate d'éthyle et les diesters en C$_6$, et moins de 4 ppm de Pd;
— une phase inférieure jaune (16,8 g) contenant le méthanol, et plus de 99,4% du sel de phosphonium et du catalyseur au palladium.

On constate qu'une séparation quasi totale du catalyseur au palladium et de tous les produits de carbonylation (légers et lourds) a été réalisée.

Le méthanol contenu dans la phase inférieure jaune est ensuite éliminé par distillation; on obtient alors un résidu homogène que l'on recycle à l'état fondu dans l'autoclave de carbonylation.

On constate qu'un recyclage direct du résidu de distillation (étapes 1b et 1c) permet de recycler la totalité du catalyseur sans élimination des produits lourds, tandis qu'un recyclage à la suite d'une opération d'extraction biphasique (étape 1e) permet de recycler le catalyseur avec une perte minimum en catalyseur et une élimination quasi totale des produits lourds.

### Exemple 2

#### 2a) *Etape de carbonylation*

Dans un autoclave de 125 cm$^3$ en alliage nickel-molybdène de marque Hastelloy B2, on introduit sous courant d'argon:
— 0,150 g (soit 0,845 mmole) de chlorure de palladium II anhydre;
— 24 g (soit 522 mmoles) d'éthanol;
— 1,54 g (soit 42,2 mmoles) d'HCl gazeux, ce qui correspond à un rapport molaire HCl/Pd de 50;

L'autoclave est fermé; on y transverse 23 g (soit 426 mmoles) de butadiène, ce qui correspond à un rapport molaire butadiène/Pd de 500 et à un rapport molaire alcool/butadiène de 1.

L'autoclave agité par secousses est porté à 120° C avec alimentation de CO à pression totale constante de 145 bars.

On laisse la réaction se dérouler pendant 2 heures à cette température. L'autoclave est ensuite refroidi à 15° C et on dégaze lentement.

Les conditions réactionnelles sont résumées dans le tableau IA.

On récupère 46,6 g d'une solution homogène jaune contenant 28% en poids d'éthanol et 56% en poids de penténoates d'éthyle.

On donne au tableau IIA les résultats de l'analyse chromatographique en phase gazeuse de la solution obtenue ainsi que les RT et RR correspondant à chaque produit formé.

Le taux de conversion global du butadiène TT est de 53,9 moles %.

L'activité spécifique A du catalyseur est de 121 h$^-$.

#### 2b) *Etape de distillation*

A un échantillon de 19,63 g de la masse réactionnelle contenant 37,9 mg (soit 0,356 matg) de Pd est ajoutée une quantité de 3 g (soit 10,17 mmoles) de chlorure de tétrabutylphosphonium, ce qui correspond à un rapport molaire onium/Pd de 28.

On effectue sur ce mélange homogène une distillation à une température de 85° C et sous une pression absolue de 3500 Pa. On recueille 11,3 g de condensat de distillation composé de 86% d'ester pentène-3 oïque (soit 9,7 g) et un résidu de 3,69 g liquide homogène, limpide de couleur rouge contenant la totalité du catalyseur au Pd et du sel de phosphonium, soit:
— 37,9 mg (soit 0,356 matg) de Pd;
— 3 g (soit 10,2 mmoles) de $PBu_4Cl$, ce qui correspond à un rapport molaire onium/Pd de 28.

### 2c) Recyclage dans l'autoclave de carbonylation

On réalise une étape de carbonylation dans les conditions décrites à l'étape 2a) à partir de:
— 3,69 g de résidu liquide de distillation
— 1,91 g (21,15 mmoles) de chloro-1 butène-2
— 12 g (261 mmoles) d'éthanol
— 14 g (259 mmoles) de butadiène, ce qui correspond à des rapports molaires butadiène/Pd = 728 et alcool/butadiène = 1.

Après deux heures de carbonylation à 120° C sous 145 bars, on récupère 27,3 g d'une solution homogène verte.

### 2d) Etape de distillation

A la totalité de la masse réactionnelle obtenue à l'étape de carbonylation 2c) (soit 27,3 g), on ajoute à nouveau 3 g (soit 10,17 mmoles) de chlorure de tétrabutylphosphonium, ce qui correspond à un rapport molaire onium/Pd de 56. On soumet le mélange à une opération de distillation dans les conditions indiquées au tableau IIIA. On recueille:

— 13,7 g de condensat de distillation composé de 56% d'ester pentène-3 oïque (soit 7,7 g);
— 7,29 g de résidu liquide homogène, limpide, de couleur orange foncé contenant:
  37,9 mg (soit 0,356 matg) de Pd;
  6 g (soit 20,3 mmoles) de chlorure de tétrabutylphosphonium.

### 2e) Recyclage dans l'autoclave de carbonylation

On réalise une étape de carbonylation dans les conditions décrites à l'étape 2a) en mettant en œuvre le résidu de distillation de l'étape 2d) et les quantités de matières premières indiquées au tableau IA.

On récupère 33,7 g d'une solution homogène verte.

### 2f) Etape de distillation

Les 33,7 g de solution verte obtenue à l'étape 2e) sont soumis à une opération de distillation dans les conditions indiquées au tableau IIIA.

On recueille 20,3 g de condensat de distillation contenant 10,2 g d'esters pentène-3 oïque et 8,20 g de résidu liquide homogène, limpide de couleur orangée brune.

### 2g) Recyclage dans l'autoclave de carbonylation

On réalise une étape de carbonylation dans les conditions indiquées à l'étape 2a) en mettant en œuvre le résidu de distillation de l'étape 2f) et les quantités de matières premières indiquées au tableau IA.

On récupère 33,7 g d'une solution homogène verte.

### 2h) Etape de distillation

Les 33,7 g de solution verte obtenue à l'étape 2g) sont soumis à une opération de distillation dans les conditions décrites au tableau IIIA.

On recueille:
— 21,2 g de condensat de distillation contenant 10,7 g d'esters pentène-3 oïque;
— 8,50 g d'un résidu liquide homogène, limpide de couleur orangé-brun.

Ce résidu de 8,50 g contient la totalité du catalyseur au Pd et du sel de phosphonium, soit:
— 37,9 mg (soit 0,356 matg) de Pd;
— 6 g (soit 20,3 mmoles) de chlorure de tétrabutylphosphonium ce qui correspond à un rapport molaire $PBu_4{}^+Cl^-/Pd$ de 56, et
— 2,4 g de produits organiques qui sont notamment des produits lourds, non volatils tels que le nonadiène-3,8 oate et les diesters en $C_6$.

### 2i) Extraction des produits lourds

On ajoute au résidu de distillation de l'étape 2h):
— 33,4 g de n-octane
— 4,4 g d'éthanol ce qui correspond à un rapport pondéral $PBu_4{}^+Cl^-$/éthanol de 1,4 et à un rapport pondéral octane/éthanol de 7,6.

Le milieu se sépare en deux phases:
— une phase supérieure incolore (33,7 g) contenant l'octane, le nonadiène-3,8 oate d'éthyle et les diesters en $C_6$ et moins de 7 ppm de Pd;
— une phase inférieure jaune visqueuse (12,6 g) contenant l'éthanol résiduel, plus de 99,6% du catalyseur au Pd et plus de 99,5% de P.

Les conditions et les résultats de cette étape d'extraction sont indiqués au tableau IV.

### 2j) Recyclage dans l'autoclave de carbonylation

On réalise une étape de carbonylation dans les conditions décrites à l'étape 2a) en mettant en œuvre les 12,6 g de phase alcoolique jaune obtenue à l'étape d'extraction 2i) et les quantités de matières premières indiquées au tableau IA.

On récupère 32,5 g de solution homogène verte claire.

### 2k) Etape de distillation

Les 32,5 g de solution vert clair obtenue à l'étape 2j) sont soumis à une opération de distillation dans les conditions indiquées au tableau IIIA.

On recueille:
— 18,7 g de condensat de distillation contenant 9,6 g d'ester pentène-3 oïque;

— 7,2 g d'un résidu liquide homogène et limpide vert contenant 0,355 matg de Pd et 20,3 mmoles de chlorure de tétrabutylphosphonium.

On constate dans la même série d'essais de recyclage 2e), 2g) et 2j), qui ont été pratiqués dans des conditions identiques:
— mêmes quantités de catalyseur et de chlorure de tétrabutylphosphonium;
— mêmes températures et pressions;
— durées de carbonylation identiques,
que la quantité de pentène-3 oate d'éthyle produite demeure environ constante et proche de 10 g ce qui signifie que *l'activité du catalyseur* est constante et ne se dégrade pas au cours des recyclages successifs.

*Exemple 3*

3a) *Etape de carbonylation*

Dans un autoclave de 125 cm³ en alliage nickel-molybdène de marque Hastelloy B2, on introduit sous courant d'argon:
— 1,725 g (soit 3,0 mmoles) de bis(dibenzal-acétone) Pd;
— 7,08 g (soit 23,9 mmoles) de chlorure de tétrabutylphosphonium ce qui correspond à un rapport molaire $PBu_4^+Cl^-/Pd$ de 8;
— 23,0 g (soit 500 mmoles) d'éthanol;
— 5,43 g (soit 60,0 mmoles) de chloro-1 butène-2, ce qui correspond à un rapport molaire HCl/Pd de 20.

L'autoclave est fermé; on y transverse 23 g (soit 426 mmoles) de butadiène, ce qui correspond à un rapport molaire butadiène/Pd de 142 et à un rapport molaire alcool/butadiène de 1,17.

L'autoclave agité par secousses est porté à 120° C avec alimentation de CO à pression totale constante de 120 bars.

On laisse la réaction se dérouler pendant 2 heures à cette température. L'autoclave est ensuite refroidi à 15° C et on dégaze lentement.

On récupère 53,9 g d'une solution homogène rouge clair contenant 23% en poids d'éthanol et 36% en poids de penténoates d'éthyle.

On donne au tableau II les résultats de l'analyse chromatographique en phase gazeuse de la solution obtenue ainsi que les RT et les RR correspondant à chaque produit formé.

Le taux de conversion global du butadiène TT est de 42,4 moles %.

L'activité spécifique A du catalyseur est de 22,4 h⁻.

3b) *Etape de distillation*

51,6 g de la solution rouge homogène et limpide, obtenue à l'étape de carbonylation 3a), sont soumis à une opération de distillation à une température de 76° C et sous une pression de 3500 Pa. On obtient:
— 28,4 g de condensat de distillation contenant 16,2 g d'esters penténoïques;
— 10,25 g de résidu liquide homogène rouge foncé contenant:
305 mg (soit 2,87 matg) de Pd,
6,8 g (soit 23 mmoles) de chlorure de tétrabutylphosphonium,

0,73 g des produits organiques non volatils constitués de nonadiène-3,8 oate de diesters en $C_6$.

3c) *Recyclage dans l'autoclave de carbonylation*

On réalise une étape de carbonylation dans les conditions décrites à l'étape 3a) en mettant en œuvre les 10,25 g de résidu liquide rouge foncé obtenu à l'étape de distillation 3b) et les quantités de matières premières indiquées au tableau IA.

On récupère 46,35 g d'une solution homogène rouge dont la composition est indiquée au tableau II.

*Exemple 4*

4a) *Etape de carbonylation*

Dans un autoclave de 125 cm³ en alliage nickel-molybdène de marque Hastelloy B2, on introduit sous courant d'argon:
— 0,1064 g (soit 0,60 mmole) de $PdCl_2$;
— 4,425 g (15 mmoles) de $PBu_4^+Cl^-$, ce qui correspond à un rapport $PBu_4Cl/Pd$ de 25;
— 18,4 g (400 mmoles) d'éthanol;
— 2,716 g (30 mmoles) de chloro-1 butène-2, ce qui correspond à un rapport molaire HCl/Pd de 50.

L'autoclave est fermé; on y transverse 21,5 g (398 mmoles) de butadiène, ce qui correspond à des rapports molaires butadiène/Pd de 713 et alcool/butadiène de 1.

L'autoclave est porté à 120° C avec alimentation de CO à pression totale constante de 130 bars.

On laisse la réaction se dérouler pendant 4 h 30 min puis l'autoclave est refroidi et on dégaze.

Les conditions réactionnelles sont résumées dans le tableau IB.

On récupère 53,1 g de solution homogène vert clair, dont l'analyse est donnée au tableau II.

Le taux de conversion global du butadiène TT est de 70,5 moles %.

L'activité spécifique A du catalyseur est de 85 h⁻¹.

4b) *Etape de distillation*

On fait subir à 50,6 g de la masse réactionnelle obtenue à l'étape de carbonylation 4a) une opération de distillation à 86° C sous 3500 Pa.

On recueille:
— 28,7 g de condensat de distillation composé de 93% de penténoates d'éthyle, et
— 7,40 g de résidu liquide homogène, limpide brun orangé contenant:
4,22 g (14,3 mmoles) de $PBu_4Cl$,
60,8 mg (0,571 matg) de Pd,
0,99 g de nonadiène-oate d'éthyle,
1,16 g de diesters en $C_6$

4c) *Recyclage dans l'autoclave de carbonylation*

On réalise une étape de carbonylation dans les conditions décrites à l'étape 4a) en mettant en œuvre les 7,40 g de résidu brun orangé de distillation de l'étape 4b) et les quantités de matières premières indiquées au tableau IB.

On récupère 40,61 g de solution homogène verte dont l'analyse est donnée au tableau II.

### 4d) *Etape de distillation*

On fait subir à 39,6 g de la solution verte obtenue à l'étape de carbonylation 4c) une étape de distillation dans les conditions indiquées au tableau IIIA.

On recueille:
— 18,80 g de condensat de distillation constitué à 86% de penténoates d'éthyle;
— 8,40 g de résidu liquide homogène rouge-brun contenant:

    4,11 g (14 mmoles) de $PBu_4Cl$
    59,3 mg (0,56 matg) de Pd.

### 4e) *Recyclage dans l'autoclave de carbonylation*

On réalise une étape de carbonylation dans les conditions décrites à l'étape 4a) en mettant en œuvre les 8,40 g de résidu liquide rouge-brun de distillation de l'étape 4d) et les quantités de matières premières indiquées au tableau IB.

Une quantité importante de $PBu_4Cl$ a été introduite (42,1 mmoles) ce afin d'avoir un rapport molaire onium/Pd de 100.

On récupère 67,2 g de solution homogène verte dont l'analyse est donnée au tableau II.

## Exemple 5

### 5a) *Etape de carbonylation*

Dans un autoclave de 125 cm³ en alliage nickel-molybdène de marque Hastelloy B2, on introduit sous courant d'argon:
— 0,0385 g (soit 0,217 mmole) de chlorure de palladium;
— 12,46 g (soit 42,25 mmoles) de chlorure de tétrabutylphosphonium ce qui correspond à un rapport molaire $PBu_4^+Cl^-/Pd$ de 200.
— 12,0 g (soit 261 mmoles) d'éthanol;
— 0,957 g (soit 10,5 mmoles) de chloro-1 butène-2, ce qui correspond à un rapport molaire HCl/Pd de 50.

L'autoclave est fermé; on y transverse 15 g (soit 278 mmoles) de butadiène, ce qui correspond à un rapport molaire butadiène/Pd de 1300 et à un rapport molaire alcool/butadiène de 0,94.

L'autoclave agité par secousses est porté à 140° C avec alimentation de CO à pression totale constante de 145 bars.

On laisse la réaction se dérouler pendant 1 h 20 min à cette température. L'autoclave est ensuite refroidi à 15° C et on dégaze lentement.

Les conditions réactionnelles sont données dans le tableau IB.

On récupère 47,8 g d'une solution homogène verte contenant 5% en poids d'éthanol et 48% en poids de penténoates d'éthyle.

On donne au tableau II les résultats de l'analyse chromatographique en phase gazeuse de la solution obtenue ainsi que les RT et RR correspondant à chaque produit formé.

Le taux de conversion global du butadiène TT est de 73,1 moles %.

L'activité spécifique A du catalyseur est de 608 h⁻.

### 5b) *Etape de distillation*

On prend 40,7 g de la masse réactionnelle obtenue à l'étape 5 a) contenant 10,6 g de chlorure de tétrabutylphosphonium, sur laquelle on effectue une opération de distillation à une température de 76° C et sous une pression de 3500 Pa.

On obtient une quantité de distillat de 22,7 g et un résidu de distillation liquide de couleur jaune orangé de 14,5 g contenant:
— 19,6 mg (soit 0,184 matg) de palladium;
— 10,6 g (soit 38 mmoles) de chlorure de tétrabutylphosphonium.

### 5c) *Recyclage dans l'autoclave de carbonylation*

On réalise une étape de carbonylation dans des conditions semblables à celles de l'étape 5a) en recyclant les 14,5 g de résidu liquide de distillation de l'étape 5b) et en mettant en œuvre les quantités de matières premières indiquées au tableau IB.

Cette étape de carbonylation est réalisée pendant 1 h à une température de 140° C et sous une pression de 145 bars.

On récupère 39,8 g d'une solution homogène verte contenant 13,8% en poids d'éthanol et 33% en poids de penténoates d'éthyle. Les résultats de l'analyse chromatographique de ladite solution sont indiqués au tableau II.

### 5d) *Etape de distillation*

On soumet 39,2 g de la masse réactionnelle obtenue à l'étape 5c) à une opération de distillation à une température de 80° C sous une pression de 3500 Pa.

On recueille:
— 22,2 g de condensat de distillation;
— 13,9 g de résidu liquide de distillation de couleur orangée contenant:

    10,4 g (37,5 mmoles) de chlorure de phosphonium
    19,6 mg (0,184 matg) de Pd
    environ 1,9 g de produits lourds.

### 5e) *Extraction des produits lourds*

On ajoute aux 13,9 g de résidu orangé de distillation de l'étape 5d), 3,6 g d'éthanol et 15 g de triméthyl-2,2,4 pentane (isooctane).

La composition ainsi obtenue contient:
— 11,1% en poids d'éthanol ce qui correspond à un rapport pondéral solvant apolaire/alcool voisin de 4,2;
— 32,0% en poids de $PBu_4Cl$, ce qui correspond à un rapport molaire $PBu_4Cl/Pd$ de 200 et un rapport pondéral $PBu_4Cl$/éthanol de 2,9.

Le milieu se sépare en deux phases:
— une phase supérieure incolore (17,9 g) contenant l'isooctane et:

    plus de 80% des produits organiques lourds,
    moins de 2,5 ppm de Pd
    moins de 50 ppm de P
— une phase inférieure jaune (14,5 g) contenant l'éthanol, 99,8% du Pd et plus de 99,8% du $PBu_4Cl$.

Les conditions et les résultats de cette étape d'extraction sont résumées au tableau IV.

La phase éthanolique jaune est ensuite recyclée vers l'autoclave de carbonylation en vue d'une nouvelle opération de carbonylation.

### Exemple 6

#### 6a) Etape de carbonylation

Dans un autoclave de 125 cm³ en alliage nickel-molybdène de marque Hastelloy B2, on introduit sous courant d'argon:
— 0,225 g (soit 1,268 mmoles) de $PdCl_2$
— 12,46 g (soit 42,25 mmoles) de chlorure de tétrabutylphosphonium ce qui correspond à un rapport molaire $PBu_4^+Cl^-$ de 33;
— 8,00 g (soit 250 mmoles) de méthanol
— 3,86 g (soit 42,2 mmoles) de chloro-1 butène-2, ce qui correspond à un rapport molaire HCl/Pd de 33.

L'autoclave est fermé; on y transverse 14 g (soit 259 mmoles) de butadiène, ce qui correspond à un rapport molaire butadiène/Pd de 205 et à un rapport molaire alcool/butadiène de 0,96.

L'autoclave agité par secousses est porté à 100° C avec alimentation de CO à pression totale constante de 120 bars. On laisse la réaction se dérouler pendant 2 heures à cette température. L'autoclave est ensuite refroidi à 15° C et on dégaze lentement.

Les conditions réactionnelles sont résumées au tableau IC.

On récupère 33,0 g d'une solution homogène verte contenant 15,4% en poids de méthanol et 38,3% en poids de penténoates de méthyle.

On donne au tableau IIA les résultats de l'analyse chromatographique en phase gazeuse de la solution obtenue ainsi que les RT et RR correspondant à chaque produit formé.

Le taux de conversion global du butadiène TT est de 45,5 moles %.

L'activité spécifique A du catalyseur est de 44 $h^{-1}$.

#### 6b) Etape de distillation

On effectue sur la masse réactionnelle de l'étape 6a) une opération de distillation à une température de 85° C sous une pression absolue de 4700 Pa. On recueille 16,9 g de condensat de distillation, contenant 12,3 g de pentène-3 oate de méthyle, et 13 g de résidu liquide orangé contenant la totalité de Pd et du sel de phosphonium.

#### 6c) Recyclage dans l'autoclave de carbonylation

On réalise une opération de carbonylation dans des conditions semblables à celle de l'étape 6a), en recyclant 12,5 g du résidu liquide orangé obtenu à l'étape de distillation 6b) et en mettant en œuvre les quantités de matières premières indiquées dans le tableau IC.

Cette étape de carbonylation est réalisée pendant 2 h à 100° C sous 120 bars.

On récupère 30,3 g d'une solution homogène verte contenant 15,7% en poids de méthanol et 36,7% en poids de penténoates de méthyle. Les résultats de l'analyse chromatographique de ladite solution sont indiqués au tableau IIA.

### Exemple 7

#### 7a) Etape de carbonylation

Dans un autoclave de 125 cm³ en alliage nickel-molybdène de marque Hastelloy B2, on introduit sous courant d'argon:
— 0,15 g (soit 0,845 mmole) de chlorure de palladium;
— 24 g (soit 522 mmoles) d'éthanol;
— 1,54 g (soit 42,2 mmoles) d'acide chlorhydrique gazeux ce qui correspond à un rapport molaire HCl/Pd de 50.

L'autoclave est fermé; on y transverse 24 g (soit 444,4 mmoles) de butadiène, ce qui correspond à un rapport molaire butadiène/Pd de 525 et à un rapport molaire alcool/butadiène de 1.

L'autoclave agité par secousses est porté à 120° C avec alimentation de CO à pression totale constante de 145 bars. On laisse la réaction se dérouler pendant 2 h à cette température. L'autoclave est ensuite refroidi à 15° C et on dégaze lentement.

Les conditions réactionnelles sont résumées au tableau IC.

On récupère 52,6 g d'une solution homogène jaune contenant 23,9% en poids de l'éthanol et 54,1% en poids de penténoates d'éthyle.

On donne au tableau IIA les résultats de l'analyse chromatographique en phase gazeuse de la solution obtenue ainsi que les RT et RR correspondant à chaque produit formé.

Le taux de conversion global du butadiène TT est de 54,3 moles %.

L'activité spécifique A du catalyseur est de 132 $h^{-1}$.

#### 7b) Etape de distillation

A 17 g de masse réactionnelle obtenue à l'étape 7a) contenant 4,05 g d'éthanol et 9,19 g d'esters penténoïques on ajoute une quantité de 8,08 g (soit 27,4 mmoles) de chlorure de tétrabutyl phosphonium ce qui correspond à un rapport molaire onium/Pd de 100.

On effectue sur ce mélange homogène une distillation à 76° C et sous une pression de 3500 Pa. On recueille 10,33 g de condensat de distillation constitué principalement d'esters penténoïques et 8,55 g de résidu visqueux jaune foncé.

Ledit résidu visqueux est une solution homogène qui prend en masse par refroidissement; il est constitué de:
— 8,08 g (27,4 mmoles) de $PBu_4Cl$;
— 29,4 mg (0,276 matg) de Pd;
— 0,42 g de produits organiques.

#### 7c) Extraction des produits lourds

On ajoute audit résidu 15 ml d'octane puis on filtre. Le résidu cristallin obtenu est ensuite lavé par 5 ml d'octane puis séché; le solide cristallin obtenu (8,15 g) est de couleur orangée; il contient 99,9% du catalyseur au palladium et du chlorure de tétrabutylphosphonium mis en œuvre; ledit solide soluble dans l'éthanol est repris pour une nouvelle étape de carbonylation.

Le filtrat octanique contient 85% du nonadiénoate d'éthyle présent dans la masse réactionnelle obtenue à l'étape 7a) et 2,1 ppm de palladium.

*Exemple 8*

8a) *Etape de carbonylation*

Dans un autoclave de 125 cm³ en alliage nickel-molybdène de marque Hastelloy B2, on introduit sous courant d'argon:
— 0,075 g (soit 0,423 mmole) de $PdCl_2$;
— 16,4 g (environ 40,6 mmoles) d'un chlorure d'onium quaternaire de marque Aliquat 336, commercialisé par General Mills, constitué principalement de chlorure de trioctylméthylammonium, ce qui correspond à un rapport molaire onium/Pd de 96 environ;
— 12 g (soit 261 mmoles) d'éthanol;
— 1,9 g (soit 21,2 mmoles) de chloro-1 butène-2, ce qui correspond à un rapport molaire HCl/Pd de 50.

L'autoclave est fermé; on y transverse 14 g (soit 259 mmoles) de butadiène, ce qui correspond à un rapport molaire butadiène/Pd de 613 et à un rapport molaire alcool/butadiène de 1.

L'autoclave agité par secousses est porté à 120° C avec alimentation de CO à pression totale constante de 145 bars. On laisse la réaction se dérouler pendant 2 h à cette température. L'autoclave est ensuite refroidi à 15° C et on dégaze lentement.

Les conditions réactionnelles sont résumées au tableau IC.

On récupère 47,5 g d'une solution homogène jaune contenant 9% en poids d'éthanol et 37% en poids de penténoates d'éthyle.

On donne au tableau IIA les résultats de l'analyse chromatographique en phase gazeuse de la solution obtenue ainsi que les RT et RR correspondant à chaque produit formé.

Le taux de conversion global du butadiène TT est de 58,9 moles %.

L'activité spécifique A du catalyseur est de 163 h⁻¹.

8b) *Etape de distillation*

On effectue sur 46,7 g de masse réactionnelle jaune obtenue à l'étape 8a) une opération de distillation à une température de 74° C et sous une pression absolue de 3500 Pa. On recueille 22,3 g de condensat de distillation, contenant 16,9 g de pentène-3-oate d'éthyle et 17,7 g de résidu liquide homogène limpide de couleur rouge contenant:
— 44,3 g (soit 0,416 matg) de Pd;
— 16,12 g (soit 40 mmoles environ) d'Aliquat 336;
— 1,07 g de produits organiques lourds non volatils qui sont essentiellement le nonadiène 3-oate d'éthyle et des diesters en $C_6$.

8c) *Recyclage dans l'autoclave de carbonylation*

On réalise une opération de carbonylation dans des conditions semblables à celles de l'étape 8a) en recyclant les 17,68 g du résidu rouge de distillation de l'étape 8b) et en mettant en œuvre les quantités de matières indiquées dans le tableau IC.

On récupère 47,66 g de masse réactionnelle jaune-vert homogène limpide dont l'analyse figure au tableau IIA.

*Exemple 9*

9a) *Etape de carbonylation*

On réalise dans les conditions décrites à l'exemple 1a) une opération de carbonylation à partir de:
— 0,1797 g (0,5 mmole, 1 matg Pd) de bis(π-allylpalladiumchlorure);
— 8,85 g (30 mmoles) de $PBu_4^+Cl^-$, ce qui correspond à un rapport molaire onium/Pd de 30;
— 18,4 g (400 mmoles) d'éthanol;
— 1,10 g (30 mmoles) de HCl, ce qui correspond à un rapport molaire HCl/Pd de 30;
— 27,2 g (400 mmoles) d'isoprène, ce qui correspond aux rapports molaires diène/Pd=400 et alcool/diène=1.

Ladite opération est réalisée à 100° C sous 200 bars pendant 4 heures.

On récupère 62,0 g d'une solution homogène jaune citron contenant:
— 9,4% en poids d'éthanol (5,8 g);
— 54% en poids d'esters β,γ-insaturés (33,75 g), constitués à 94% de méthyl-4 pentène-3 oate et à 6% de méthyl-3, pentène-3 oate et de diméthyl-2,3, butène-3 oate d'éthyle.

Le taux de conversion partiel RR en esters β,γ-insaturés est de 59,4 moles %.

L'activité spécifique A du catalyseur, exprimée en méthyl-4 pentène-3 oate d'éthyle est de 56 h⁻¹.

9b) *Etape de distillation*

On fait subir à 29,8 g de la masse réactionnelle de l'étape 9a) une opération de distillation à une température de 90° C sous une pression absolue de 3500 Pa.

On obtient:
— 18,0 g de condensat de distillation contenant 16 g d'esters β,γ-insaturés (soit 88,8% en poids);
— 5,2 g de résidu de distillation, liquide, homogène, limpide de couleur rouge contenant:
— 4,25 g (14,4 mmoles) de $PBu_4Cl$;
— 51 mg (0,48 matg) de Pd;
— 0,84 g de produits organiques.

9c) *Etape de recyclage dans l'autoclave de carbonylation*

On réalise une étape de carbonylation dans les conditions décrites en 9a) à partir de:
— 5,2 g de résidu liquide de distillation obtenu à l'étape 9b);
— 9,2 g (200 mmoles) d'éthanol;
— 526 mg (14,4 mmoles) de HCl;
— 13,1 g (192 mmoles) d'isoprène.

Après 4 heures de carbonylation à 100° C sous 200 bars on récupère 31,16 g d'une solution jaune, limpide, homogène, contenant 19,8 g (soit 63% en poids) d'esters β,γ-insaturés constitués de 94% de méthyl-4 pentène-3 oate d'éthyle.

La taux de conversion partiel RR en esters β,γ-insaturés est de 72,6 moles %.

L'activité spécifique A du catalyseur, exprimée en méthyl-4 pentène-3 oate d'éthyle est de 72 h⁻¹.

### 9d) *Deuxième étape de distillation*

On fait subir à 27 g de la masse réactionnelle obtenue à l'étape 9c) une opération de distillation à une température de 82° C sous 2800 Pa.

On recueille:
— 19,1 g de condensat de distillation contenant 16,9 g (soit 88,5% en poids) d'esters β,γ-insaturés;
— 5,8 g de résidu de distillation, liquide, homogène, rouge-brun et contenant:
  3,7 g (12,5 mmoles) de PBu₄Cl,
  44,2 mg (0,415 matg) de Pd,
  2,0 g de produits organiques.

### 9e) *Extraction des produits lourds*

On ajoute au résidu de distillation de l'étape 9d) 0,215 g d'éthanol; on obtient un liquide visqueux rouge-brun, auquel on ajoute 8,8 g de n-dodécane à une température de 50° C.

La composition ainsi obtenue contient:
— 1,5 g en poids d'éthanol, ce qui correspond à un rapport pondéral alcane/alcool de 41;
— 25% en poids de PBu₄Cl, ce qui correspond à un rapport molaire onium/Pd=30 et un rapport pondéral onium/alcool 17.

Après agitation, refroidissement à 10° C et décantation, le milieu se sépare en deux phases:
— une phase supérieure incolore de 10,4 g constituée de n-dodécane ayant solubilisé 1,6 g de produits organiques, contenant moins de 3 ppm de Pd et moins de 20 ppm de phosphore;
— une phase inférieure très visqueuse, brun rougeâtre, de 4,4 g contenant l'éthanol et plus de 99,9% de palladium et du sel de phosphonium.

Les conditions et les résultats de cette étape d'extraction sont résumés au tableau IV.

La phase inférieure brun rougeâtre est recyclée vers l'autoclave de carbonylation en vue d'une nouvelle opération de carbonylation.

### Exemple 10

#### 10a) *Etape de carbonylation*

Dans un autoclave de 125 cm³ en alliage nickel-molybdène de marque Hastelloy B2, on introduit sous courant d'argon:

— 287,5 mg (soit 0,5 mmole) de bis(dibenzal-acétone) Pd;
— 15,4 g (soit 20 mmoles) de bromure de tétradodécylammonium ce qui correspond à un rapport molaire NDod₄Br/Pd de 40;
— 12 g (soit 261 mmoles) d'éthanol;
— 2,7 g (soit 20 mmoles) de bromo-1 butène-2, ce qui correspond à un rapport molaire HBr/Pd de 40.

L'autoclave est fermé; on y transverse 13 g (soit 241 mmoles) de butadiène, ce qui correspond à un rapport molaire butadiène/Pd de 482 et à un rapport molaire alcool/butadiène de 1,08.

L'autoclave agité par secousses est porté à 100° C avec alimentation de CO à pression totale constante de 145 bars.

On laisse la réaction se dérouler pendant 1 h 30 min à cette température, puis pendant 2 h 10 min à 120° C. L'autoclave est ensuite refroidi à 15° C et on dégaze lentement.

On récupère 30,8 g d'une solution homogène orange contenant 18,5% en poids d'éthanol et 15,6% en poids de penténoates d'éthyle.

On donne au tableau V les résultats de l'analyse chromatographique en phase gazeuse de la solution obtenue ainsi que les RT et les RR correspondant à chaque produit formé.

Le taux de conversion global du butadiène TT est de 25 moles %.

L'activité spécifique A du catalyseur est de 19 h⁻¹.

#### 10b) *Etape de distillation* (résumée au tableau IIIC)

Les 30,8 g de la solution orange homogène et limpide, obtenue à l'étape de carbonylation 10a), sont soumis à une opération de distillation à une température de 93° C et sous une pression de 3500 Pa environ. On obtient:

— 11,7 g de condensat de distillation contenant 2,96 g d'esters penténoïques;
— 16,6 g de résidu liquide homogène rouge vif contenant 0,5 matg de Pd, 20 mmoles de bromure de tétradodécylammonium et 0,9 g des produits organiques non volatils constitués de nonadiène-3,8 oate et de diesters en C₆.

#### 10c) *Recyclage dans l'autoclave de carbonylation*

On réalise une étape de carbonylation dans les conditions décrites à l'étape 10a) en mettant en œuvre les 16,6 g de résidu liquide rouge foncé obtenu à l'étape de distillation 10b) et les quantités suivantes de réactifs:
— 2,7 g (soit 20 mmoles) de bromo-1 butène-2;
— 12 g (soit 261 mmoles) d'éthanol;
— 13,5 g (soit 250 mmoles) de butadiène, ce qui correspond aux rapports molaires butadiène/palladium de 500 et alcool/butadiène de 1,04.

L'opération est réalisée pendant 1 h à 100° C et 3 h à 120° C avec alimentation de CO à pression totale constante de 145 bars.

On récupère 34,6 g d'une solution orangée homogène contenant 20,3% en poids d'éthanol et 15,5% en poids de penténoates d'éthyle.

On donne au tableau V les résultats de l'analyse chromatographique en phase gazeuse de la solution obtenue ainsi que les RT et RR correspondant à chaque produit formé.

Le taux de conversion globale du butadiène TT est de 25,5 moles %; l'activité spécifique A du catalyseur est de 21 h⁻¹.

## Exemple 11

### 11a) *Etape de carbonylation*

Dans un autoclave de 125 cm$^3$ en alliage nickel-molybdène de marque Hastelloy B2, on introduit sous courant d'argon:
— 75 mg (soit 0,423 mmole) de PdCl$_2$;
— 12,46 g (42,3 mmoles) de PBu$_4^+$Cl$^-$, ce qui correspond à un rapport PBu$_4$Cl/Pd de 100;
— 12 g (261 mmoles) d'éthanol;
— 1,912 g (21,2 mmoles) de chloro-1 butène-2, ce qui correspond à un rapport molaire HCl/Pd de 50.

L'autoclave est fermé; on y transverse 13,5 g (250 mmoles) de butadiène, ce qui correspond à des rapports molaires butadiène/Pd de 591 et alcool/butadiène de 1,04.

L'autoclave est porté à 120° C avec alimentation de CO à pression totale constante de 145 bars.

On laisse la réaction se dérouler pendant 4 h puis l'autoclave est refroidi et on dégaze.

Les conditions réactionnelles sont résumées dans le tableau ID.

On récupère 43,4 g de solution homogène vert clair, dont l'analyse est donnée au tableau IIB.

Le taux de conversion global du butadiène TT est de 83,6 moles %.

L'activité spécifique A du catalyseur est de 110 h$^{-1}$.

### 11b) *Etape de distillation* (résumée au tableau IIIC)

On fait subir à 42,24 g de la masse réactionnelle obtenue à l'étape de carbonylation 11a) une opération de distillation à 80° C sous 3500 Pa.

On recueille:
— 23,8 g de condensat de distillation composé de 22,6 g de penténoates d'éthyle, et
— 15,1 g de résidu liquide homogène, limpide rouge contenant:

41,2 mmoles de PBu$_4$Cl,
0,41 matg de Pd,
0,32 g de nonadiène-3,8-oate d'éthyle,
2,01 g de diesters en C$_6$.

### 11c) *Extraction des produits lourds* (résumée au tableau IV)

On ajoute au résidu de distillation de l'étape 11b):

3 g d'eau,
36,2 g de n-dodécane, ce qui correspond à un rapport pondéral PBu$_4^+$Cl$^-$/eau de 4,04 et à un rapport pondéral dodécane/eau de 12.

Le milieu se sépare en deux phases:
— une phase supérieure incolore (37,7 g) contenant le dodécane et environ 62% du nonadiène-3,8 oate d'éthyle et 67% des diesters en C$_6$, moins de 5,5 ppm de Pd et moins de 23 ppm de P;
— une phase inférieure rouge (16,2 g) contenant l'eau, plus de 99,4% du catalyseur au Pd et plus de 99,9% de PBu$_4$Cl.

### 11d) *Elimination de l'eau*

La phase inférieure rouge est chauffée à 90° C sous 3500 Pa jusqu'à élimination totale de l'eau.

On recueille 13,2 g d'un résidu contenant 0,411 matg de Pd et 41,1 mmoles de P que l'on recycle à l'état fondu dans l'autoclave de carbonylation.

### 11e) *Carbonylation*

On réalise une étape de carbonylation dans les conditions indiquées à l'étape 11a) en mettant en œuvre les 13,2 g de résidu et les quantités suivantes de réactifs:
— 1,86 g (soit 20,55 mmoles) de chloro-1 butène-2;
— 12 g (soit 261 mmoles) d'éthanol;
— 13,5 g (soit 250 mmoles) de butadiène, ce qui correspond aux rapports molaires suivants: HCl/Pd=50, butadiène/Pd=608, alcool/butadiène=104.

L'opération est réalisée à 120° C pendant 2 heures avec alimentation de CO à pression totale constante de 145 bars.

On récupère 42,8 g d'une solution verte homogène contenant 3,1% en poids d'éthanol et 49,4% en poids de penténoates d'éthyle.

Le résultat de l'analyse chromatographique en phase gazeuse est donné au tableau IIB.

Le taux de conversion globale du butadiène TT est de 72,9 moles %, l'activité spécifique A du catalyseur est de 201 h$^{-1}$.

## Exemple 12

### 12a) *Etape de carbonylation*

Cette étape a été décrite à l'exemple 4e); la masse réactionnelle obtenue est de 67,2 g et se présente sous la forme d'une solution homogène verte.

### 12b) *Etape de distillation* (résumée au tableau IIIC)

On prend 62,9 g de cette masse réactionnelle auxquels on ajoute 16,52 g de PBu$_4$Cl et 98,8 mg de PdCl$_2$.

La composition obtenue contient 1,077 matg de Pd et 108,3 mmoles de PBu$_4$Cl.

On fait subir à cette composition une opération de distillation à 82° C sous 3500 Pa environ.

On recueille:
— 33,7 g de condensat de distillation contenant 26,2 g de penténoates d'éthyle, et
— 42,10 g d'un résidu liquide homogène brun orangé contenant:

1,077 matg de Pd,
108,3 mmoles de PBu$_4$Cl,
0,83 g de nonadiénoate d'éthyle,
3,75 g de diesters en C$_6$.

### 12c) *Extraction des produits lourds* (résumée au tableau IV)

On prend 9 g de résidu brun orangé (ce qui correspond à 0,230 matg de Pd et 23,15 mmoles de PBu$_4$Cl) auxquels on ajoute 13 g d'acétonitrile et 40 g de n-octane, ce qui correspond à:
— un rapport pondéral onium/solvant polaire de 0,53;
— un rapport pondéral n-octane/solvant polaire de 3,1;
— un rapport molaire onium/Pd de 100.

Le milieu se sépare en deux phases:

une phase supérieure incolore de 39,8 g contenant le n-octane, plus de 60% des produits organiques lourds, moins de 1,5 ppm de Pd et moins de 30 ppm de P;

une phase inférieure orange de 21,4 g contenant l'acétonitrile, plus de 99,7% de Pd et plus de 99,8% de P.

### 12d) *Elimination de l'acétonitrile*

La phase inférieure orange est soumise à une opération de distillation à 70° C sous 3500 Pa jusqu'à élimination complète de l'acétonitrile.

On récupère un liquide visqueux brun orangé homogène contenant 0,230 matg de Pd et 23,1 mmoles de $PBu_4Cl$, que l'on recycle à l'état fondu dans l'autoclave de carbonylation.

### 12e) *Etape de carbonylation*

On introduit en outre dans l'autoclave de carbonylation:
— 7 g (soit 152 mmoles) d'éthanol;
— 1,04 g (soit 11,5 mmoles) de chloro-1 butène-2, et
— 10 g (soit 185 mmoles) de butadiène, ce qui correspond aux rapports molaires HCl/Pd=50, butadiène/Pd=805, alcool/butadiène=0,82.

L'opération de carbonylation est réalisée pendant 2 h à 120° C avec alimentation de CO à pression totale constante de 145 bars. Les conditions réactionnelles sont résumées au tableau ID.

On obtient 27,8 g de solution verte homogène, dont l'analyse est donnée au tableau IIB.

Le taux de conversion globale du butadiène TT est de 56,4 moles %. L'activité spécifique du catalyseur A est de 206 $h^{-1}$.

### Exemple 13

13a) *Extraction des produits lourds* (résumée au tableau IV)

On prend 21,5 g du résidu de distillation obtenu à l'exemple 12b), ce qui correspond à 0,55 matg de Pd et 55,4 mmoles de $PBu_4Cl$.

On y ajoute 27 g de N,N-diméthylformamide et 81 g de cyclohexane, ce qui correspond à:
— un rapport pondéral onium/solvant polaire de 0,61;
— un rapport pondéral solvant apolaire/solvant polaire de 3;
— un rapport molaire onium/Pd de 100.

Le milieu se sépare en deux phases:
— une phase supérieure incolore de 86,8 g contenant le cyclohexane, plus de 80% des produits organiques lourds, moins de 3 ppm de Pd et moins de 65 ppm de P;
— une phase inférieure brune de 48,4 g contenant le diméthylformamide, plus de 99,5% du Pd et plus de 99,5% de P.

### 13b) *Elimination du N,N-diméthylformamide*

La phase inférieure brune est soumise à une opération de distillation à 80° C sous 3500 Pa environ jusqu'à élimination totale du diméthylformamide.

On récupère 18,20 g d'un résidu brun clair homogène constitué de 0,55 matg de Pd et 55,4 mmoles de $PBu_4Cl$, que l'on recycle à l'état fondu dans l'autoclave de carbonylation.

### 13c) *Etape de carbonylation*

On introduit en outre dans l'autoclave de carbonylation:
— 12 g (soit 261 mmoles) d'éthanol;
— 2,49 g (soit 27,5 mmoles) de chloro-1 butène-2;
— 14 g (soit 259 mmoles) de butadiène, ce qui correspond aux rapports molaires HCl/Pd=50, butadiène/Pd de 470 et alcool/butadiène de 1 environ.

L'opération de carbonylation est réalisée pendant 2 heures à 120° C avec alimentation de CO à pression totale constante de 145 bars. Les conditions de carbonylation sont résumées au tableau ID. On récupère 50,4 g de masse réactionnelle verte homogène, dont l'analyse est donnée au tableau IIB.

Le taux de conversion globale du butadiène TT est de 76,5%. L'activité spécifique A du catalyseur est de 165 $h^{-1}$.

### Exemple 14

*Exemple comparatif avec NBu₄Cl*

Cet exemple est donné à titre comparatif en utilisant comme sel d'onium quaternaire du chlorure d'ammonium quaternaire non fusible (décomposition au-dessus de 200° C).

### 14a) *Etape de carbonylation*

On réalise dans les conditions décrites à l'exemple 1a une opération de carbonylation à partir de:
— 14,5 g (268,5 mmoles) de butadiène;
— 0,0750 g (0,423 mmole) de $PdCl_2$;
— 12 g (261 mmoles) d'éthanol;
— 1,914 g (21,15 mmoles) de chloro-1 butène-2;
— 11,75 g (42,25 mmoles) de $NBu_4Cl$.

Ladite opération est réalisée à 120° C et sous une pression de 145 bars pendant 2 heures.

On récupère 44 g d'une solution homogène verte contenant 48% de pentène-3 oate d'éthyle, ce qui correspond à une activité spécifique de 195 $h^{-1}$.

### 14b) *Etape de distillation*

On fait subir à 36,2 g de la masse réactionnelle une opération de distillation à une température de 80° C et sous une pression absolue de 3500 Pa.

On constate que le résidu de distillation trouble marron n'est pas un liquide homogène mais prend en masse et cristallise sur les parois de l'appareillage de distillation; ledit résidu n'est donc pas directement recyclable.

### Exemple 15

Cet exemple est donné à titre comparatif en remplaçant à l'étape de distillation le chlorure d'onium quaternaire fusible par un solvant organique non volatil dans les conditions de l'opération de distillation.

15a) *Etape de carbonylation*

On réalise l'étape de carbonylation décrite à l'exemple 6a) dans des conditions identiques.

Les résultats de l'opération de carbonylation sont identiques.

15b) *Etape de distillation*

A la masse réactionnelle obtenue (53 g) on ajoute 24,2 g de méthyl-2 glutarate de diéthyle.

On réalise une opération de distillation à une température de 90° C et sous une pression absolue de 3700 Pa.

On constate que le résidu de distillation brun foncé obtenu est trouble et contient du palladium métallique solide en suspension, difficilement recyclable industriellement.

En outre, contrairement au procédé faisant l'objet de la présente invention, la séparation entre les produits lourds et le catalyseur au palladium ne peut être réalisée d'une manière simple et efficace, ce qui rend toute application industrielle aléatoire en raison de l'accumulation progressive des produits lourds non séparables.

Tableau I A

| Ex. | BD (mM) | ROH (mM) | Catalyseur (matg Pd) | Cocatalyseur (mM) | BD/Pd molaire | HCl/Pd molaire | Onium (mM) | Onium/Pd molaire | T (°C) | Durée (h) | Pco (bars) | MR Poids (g) | MR Couleur |
|---|---|---|---|---|---|---|---|---|---|---|---|---|---|
|  |  | EtOH | $[\langle(Pd-Cl]_2$ | ⌇Cl |  |  | $PBu_4Cl$ |  |  |  |  |  |  |
| 1a | 269 | 261 | 0,423 | 2,1 | 636 | 5 | 42,2 | 100 | 120 | 2 | 196 | 41,17 | jaune-vert |
| 1c | 111 | 130 | 0,206 | 1,03 | 540 | 5 | 20,5 | 100 | 120 | 2 | 196 | 19,02 | jaune-vert |
| 2a | 426 | 522 | $PdCl_2$ 0,845 | HCl 42,2 | 500 | 50 | 0 | 0 | 120 | 2 | 145 | 46,6 | jaune |
|  |  |  |  | ⌇Cl |  |  |  |  |  |  |  |  |  |
| 2c | 259 | 261 | 0,356 | 21,1 | 728 | 60 | 10,17 | 28 | 120 | 2 | 145 | 27,3 | vert |
| 2e | 296 | 261 | 0,356 | 21,1 | 830 | 60 | 20,34 | 56 | 120 | 2 | 145 | 33,7 | vert |
| 2g | 296 | 261 | 0,356 | 21,1 | 830 | 60 | 20,34 | 56 | 120 | 2 | 145 | 33,3 | vert |
| 2j | 278 | 261 | 0,355 | 21,1 | 780 | 60 | 20,24 | 56 | 120 | 2 | 145 | 32,5 | vert |

Tableau I B

| Ex. | BD (mM) | ROH (mM) | Catalyseur (matg Pd) | Cocatalyseur (mM) | BD/Pd molaire | HCl/Pd molaire | Onium (mM) | Onium/Pd molaire | T (°C) | Durée (h) | Pco (bars) | MR Poids (g) | MR Couleur |
|---|---|---|---|---|---|---|---|---|---|---|---|---|---|
|  |  | EtOH | $Pd (DBA)_2$ | ⌇Cl |  |  | $PBu_4Cl$ |  |  |  |  |  |  |
| 3a | 426 | 500 | 3,0 | 60 | 142 | 20 | 23,9 | 8 | 120 | 2 | 120 | 53,9 | rouge |
| 3c | 426 | 500 | 2,87 | 60 | 148 | 20 | 23 | 8 | 120 | 2 | 120 | 46,35 | rouge |
| 4a | 398 | 400 | $PdCl_2$ 0,60 | 30 | 713 | 50 | 15 | 25 | 120 | 4,5 | 130 | 53,1 | vert clair |
| 4c | 370 | 400 | 0,571 | 30 | 650 | 52,6 | 14,3 | 25 | 120 | 2 | 145 | 40,61 | vert |
| 4e | 361 | 400 | 0,56 | 30 | 645 | 53,5 | 13,39+42,07 | 100 | 120 | 2 | 145 | 67,2 | vert |
| 5a | 278 | 261 | 0,217 | 10,5 | 1313 | 50 | 42,2 | 200 | 140 | 1,37 | 145 | 47,8 | vert |
| 5c | 278 | 261 | 0,184 | 10,5 | 1510 | 56 | 38 | 200 | 140 | 1 | 145 | 39,8 | vert |

Tableau I C

| Ex. | BD (mM) | ROH (mM) | Catalyseur (matg Pd) | Cocatalyseur (mM) | Diène/Pd | HCl/Pd | Onium (mM) | Onium/Pd molaire | T (°C) | Durée (h) | Pco (bars) | MR Poids (g) | MR Couleur |
|---|---|---|---|---|---|---|---|---|---|---|---|---|---|
| | | MeOH | Pd Cl$_2$ | ⌇Cl | | | PBu$_4$Cl | | | | | | |
| 6a | 259 | 250 | 1,268 | 42,2 | 205 | 33 | 42,2 | 33 | 100 | 2 | 120 | 33 | vert |
| 6c | 268 | 250 | 1,222 | 40,3 | 220 | 33 | 40,7 | 33 | 100 | 2 | 120 | 30,3 | vert |
| 7a | 444 | EtOH 522 | 0,845 | HCl 42,2 | 525 | 50 | 0 | 0 | 120 | 2 | 145 | 52,6 | jaune |
| | | | | ⌇Cl | | | Aliquat | | | | | | |
| 8a | 259 | 261 | 0,423 | 21,1 | 612 | 50 | 40,6 | 96 | 120 | 2 | 145 | 47,5 | jaune |
| 8c | 268 | 261 | 0,416 | 21,1 | 644 | 50 | 40 | 96 | 120 | 2 | 145 | 47,6 | jaune |
| | | | [<(PdCl]$_2$ | HCl | | | PBu$_4$Cl | | | | | | |
| 9a | 400 | 400 | 1,0 | 30 | 400 | 30 | 30 | 30 | 100 | 4 | 200 | 62,0 | jaune citron |
| 9c | 192 | 200 | 0,48 | 14,4 | 400 | 30 | 14,4 | 30 | 100 | 4 | 200 | 31,2 | jaune vert |

Tableau I D

| Ex. | BD (mM) | ROH (mM) | Catalyseur (matg Pd) | Cocatalyseur (mM) | Diène/Pd | HCl/Pd | Onium (mM) | Onium/Pd molaire | T (°C) | Durée (h) | Pco (bars) | MR Poids (g) | MR Couleur |
|---|---|---|---|---|---|---|---|---|---|---|---|---|---|
| | | EtOH | Pd-Cl$_2$ | ⌇Cl | | | PBu$_4$Cl | | | | | | |
| 11a | 250 | 261 | 0,423 | 21,1 | 591 | 50 | 42,3 | 100 | 120 | 4 | 145 | 43,4 | vert |
| 11e | 250 | 261 | 0,411 | 20,55 | 608 | 50 | 41,1 | 100 | 120 | 2 | 145 | 42,8 | vert |
| 12e | 185 | 152 | 0,230 | 11,5 | 805 | 50 | 23,1 | 100 | 120 | 2 | 145 | 27,8 | vert |
| 13c | 259 | 261 | 0,55 | 27,5 | 470 | 50 | 55,4 | 100 | 120 | 2 | 145 | 50,4 | vert |

0 075 524

Tableau II A

| Ex. | TT (%) | A (h−1) | P3+4 | | P′ | | C9 | | HC8 | | ROC4 | | C6 | | RR Cl (%) | ROH résiduel (g) |
|---|---|---|---|---|---|---|---|---|---|---|---|---|---|---|---|---|
| | | | RT (%) | RR (%) | RT (%) | RR (%) | RT (%) | RR (%) | RT (%) | RR (%) | RT (%) | RR (%) | RT (%) | RR (%) | | |
| 1a | 80,7 | 232 | 90,8 | 73,3 | 1,9 | 1,5 | 1,4 | 1,1 | 0,7 | 0,6 | ε | ε | 2,0 | 1,6 | 0,8 | 2,15 |
| 1c | 86,5 | 221 | 94,5 | 81,7 | 1,7 | 1,4 | 0,3 | 0,3 | 0,2 | 0,2 | ε | ε | 2,4 | 2,1 | 0,4 | 0,9 |
| 2a | 53,9 | 121 | 90 | 48,5 | 2,9 | 1,6 | 1,2 | 0,7 | 2,1 | 1,3 | 1,8 | 0,9 | 1,0 | 0,6 | 1,6 | 13,1 |
| 3a | 42,4 | 22,4 | 83,7 | 35,5 | 2,0 | 0,8 | 2,4 | 1 | 4,8 | 2 | 1 | 0,4 | 0,1 | 0,4 | 6,4 | 12,5 |
| 3c | 26,1 | 15,8 | 81,2 | 21,3 | 2,2 | 0,6 | 1,7 | 0,4 | 9,3 | 2,4 | 0,4 | 0,1 | 1,2 | 0,3 | 3,9 | 13,5 |
| 4a | 70,5 | 85 | 81,6 | 57,5 | 1,6 | 1,1 | 7,3 | 5,2 | 3,8 | 2,7 | ε | ε | 3,1 | 2,2 | 4,6 | 4,44 |
| 4c | 40,2 | 112 | 85,5 | 31,8 | 2,2 | 0,8 | 3,6 | 1,5 | 3,8 | 1,4 | ε | ε | 3,6 | 1,5 | 3,3 | 10,97 |
| 4e | 74,9 | 220 | 91,0 | 68,2 | 1,9 | 1,4 | 1,1 | 0,8 | 2,0 | 1,5 | ε | ε | 2,7 | 2,0 | 3,7 | 4,99 |
| 5a | 73,1 | 608 | 88,7 | 64,9 | 1,8 | 1,3 | 2,6 | 1,9 | 2,2 | 1,6 | ε | ε | 2,9 | 2,1 | 6,0 | 2,4 |
| 5c | 42,3 | 570 | 87,2 | 36,9 | 2,7 | 2,3 | 1,6 | 0,7 | 7 | 3 | ε | ε | 1 | 0,4 | 2,6 | 6,5 |
| 6a | 45,5 | 44 | 93,9 | 42,7 | 1,5 | 0,7 | 0,9 | 0,4 | 2,7 | 1,2 | ε | ε | 0,6 | 0,2 | — | 5,1 |
| 6c | 39,6 | 40 | 92,0 | 36,4 | 1,8 | 0,7 | 1,3 | 0,5 | 2,5 | 1,0 | 0,3 | 0,1 | 0,8 | 0,3 | — | 4,7 |
| 7a | 54,3 | 132 | 92,0 | 50 | 2,2 | 1,2 | 1,3 | 0,7 | 2,8 | 1,5 | 1,4 | 0,8 | 0,2 | 0,1 | 3,2 | 12,6 |
| 8a | 58,9 | 163 | 90,1 | 53,1 | 1,5 | 0,9 | 1,5 | 0,9 | 2,2 | 1,3 | ε | ε | 2,9 | 1,7 | 5,9 | 4,3 |
| 8c | 55,5 | 157 | 87,2 | 48,7 | 1,6 | 0,9 | 3,5 | 2 | 2,3 | 1,3 | ε | ε | 2,8 | 1,6 | 6,1 | 4,2 |

Tableau II B

| Ex. | TT (%) | A (h−1) | P3+4 | | P′ | | C9 | | HC8 | | ROC4 | | C6 | | RR Cl (%) | ROH résiduel (g) |
|---|---|---|---|---|---|---|---|---|---|---|---|---|---|---|---|---|
| | | | RT (%) | RR (%) | RT (%) | RR (%) | RT (%) | RR (%) | RT (%) | RR (%) | RT (%) | RR (%) | RT (%) | RR (%) | | |
| 11a | 83,6 | 110 | 88,8 | 74,2 | 1,2 | 1,0 | 1,8 | 1,5 | 0,6 | 0,5 | ε | ε | 5,3 | 4,4 | 3,0 | 0,04 |
| 11e | 72,9 | 201 | 90,6 | 66,1 | 1,5 | 1,2 | 0,9 | 0,7 | 0,9 | 0,7 | ε | ε | 3,8 | 2,8 | 3,6 | 1,33 |
| 12e | 56,4 | 206 | 91,1 | 51,3 | 1,9 | 1,1 | 1,2 | 0,7 | 0,8 | 0,5 | ε | ε | 3,0 | 1,7 | 2,6 | 0,73 |
| 13c | 76,5 | 165 | 91,4 | 69,9 | 1,5 | 1,1 | 1,2 | 0,9 | 1,0 | 0,8 | ε | ε | 1,6 | 1,2 | 2,8 | 0,8 |

*Tableau III A*

| Ex. | MR (g) | PBu$_4$Cl (mM) | Onium / Pd molaire | T (°C) | P (Pa) | Résidu de distillation | | | | Condensat | |
|---|---|---|---|---|---|---|---|---|---|---|---|
| | | | | | | g couleur | Pd matg | onium (mM) | produits organiques | g | P$_{3+4}$ g |
| 1b | 20 | 20,5 | 100 | 84 | 3500 | 7,5 orange | 0,206 | 20,5 | 1,5 | 10,8 | 10,3 |
| 1d | 15,1 | 15,4 | 100 | 84 | 3100 | 4,95 orange | 0,155 | 15,4 | 0,4 | 8,4 | |
| 2b | 19,6 | 10,2 | 28 | 85 | 3500 | 3,69 rouge | 0,356 | 10,2 | 0,6 | 11,3 | 9,7 |
| 2d | 27,3 | 10,17×2 | 56 | 80 | 3500 | 7,29 orange | 0,356 | 20,3 | 1,23 | 13,7 | 7,7 |
| 2f | 33,7 | 20,3 | 56 | 83 | 3500 | 8,2 | 0,356 | 20,3 | 2,14 | 20,3 | 10,2 |
| 2h | 33,3 | 20,3 | 56 | 82 | 3500 | 8,5 | 0,356 | 20,3 | 2,4 | 21,2 | 10,7 |
| 2k | 32,5 | 20,2 | 56 | 82 | 3500 | 7,2 vert | 0,355 | 20,2 | 1,1 | 18,7 | 9,6 |
| 3b | 51,6 | 23 | 8 | 76 | 3500 | 10,25 rouge | 2,87 | 23 | 1,8 | 28,4 | 16,2 |
| 4b | 50,6 | 14,3 | 25 | 86 | 3500 | 7,4 | 0,571 | 14,3 | 3,0 | 28,7 | 26,7 |
| 4d | 39,6 | 14 | 25 | 80 | 3500 | 8,4 | 0,56 | 14 | 4,2 | 18,8 | 16,2 |

*Tableau III B*

| Ex. | MR (g) | Onium (mM) | Onium / Pd molaire | T (°C) | P (Pa) | Résidu de distillation | | | | Condensat | |
|---|---|---|---|---|---|---|---|---|---|---|---|
| | | | | | | g couleur | Pd matg | onium (mM) | produits organiques | g | $P_{3+4}$ g |
| 5b | 40,7 | PBu$_4$Cl 38 | 200 | 76 | 3500 | 14,5 jaune orange | 0,184 | 38 | 3,8 | 22,7 | 19,5 |
| 5d | 39,2 | 37,5 | 200 | 80 | 3500 | 13,9 jaune orange | 0,184 | 37,5 | 2,5 | 22,2 | |
| 6b | 33 | 42,2 | 33 | 85 | 4700 | 13,0 orange | 1,268 | 42,2 | 0,3 | 16,9 | 12,3 |
| 7b | 17 | 27,4 | 100 | 76 | 3500 | 8,55 jaune foncé | 0,276 | 27,4 | 0,4 | 10,3 | 9,1 |
| 8b | 46,7 | Aliquat 40 | 96 | 74 | 3500 | 17,7 rouge | 0,416 | 40 | 1,1 | 22,3 | 16,9 |
| 9b | 29,8 | PBu$_4$Cl 14,4 | 30 | 90 | 3500 | 5,2 rouge | 0,48 | 14,4 | 0,84 | 18,0 | 16,0 |
| 9d | 27,0 | 12,5 | 30 | 82 | 2800 | 5,8 rouge-brun | 0,42 | 12,5 | 2,0 | 19,1 | 16,9 |

*Tableau III C*

| Ex. | MR (g) | Onium (mM) | Onium / Pd molaire | T (°C) | P (Pa) | Résidu de distillation | | | | Condensat | |
|---|---|---|---|---|---|---|---|---|---|---|---|
| | | | | | | g couleur | Pd matg | onium (mM) | produits organiques | g | $P_{3+4}$ g |
| 10b | 30,8 | NDod$_4$Br 20 | 40 | 93 | 3500 | 16,6 rouge | 0,5 | 20 | 0,9 | 11,7 | 2,96 |
| 11b | 42,24 | PBu$_4$Cl 41,2 | 100 | 80 | 3500 | 15,1 rouge | 0,41 | 41,2 | 2,33 | 23,8 | 22,6 |
| 12b | 62,9 | 108,3 | 100 | 82 | 3500 | 42,1 orange | 1,077 | 108,3 | 4,58 | 33,7 | 26,2 |

0 075 524

Tableau IV

| Ex. | Onium / Pd molaire | Alcane (g) | Polaire (g) | Alcane polaire pondéral | Onium polaire pondéral | Phase polaire | | | Phase alcanique | | |
|---|---|---|---|---|---|---|---|---|---|---|---|
| | | | | | | g | % récupérés de Pd | onium | g | % récupérés de C9+C6 | Pd (ppm) |
| 1e | 100 | n-hexane 40 | MeOH 13 | 3,1 | 0,35 | 16,8 | >99,4 | >99,4 | 41,1 | | <4 |
| 2i | 56 | n-octane 33,4 | EtOH 4,4 | 7,6 | 1,4 | 12,6 | >99,6 | >99,5 | 33,7 | | <7 |
| 5e | 200 | isooctane 15 | EtOH 3,6 | 4,2 | 2,9 | 14,5 | >99,8 | >99,8 | 17,9 | >80 | <2,5 |
| 9e | 30 | n-dodécane 8,8 | EtOH 0,215 | 41 | 17 | 4,4 | >99,9 | >99,9 | 10,4 | >90 | <3 |
| 11c | 100 | n-dodécane 36,2 | eau 3 | 12 | 4,04 | 16,26 | >99,4 | >99,9 | 37,7 | >65 | <5,5 |
| 12c | 100 | n-octane 40 | AN 13 | 3,1 | 0,53 | 21,4 | >99,7 | >99,8 | 39,8 | >60 | <1,5 |
| 13a | 100 | cyclohexane 81 | DMF 27 | 3 | 0,61 | 48,4 | >99,5 | >99,5 | 86,8 | >80 | <3 |

Tableau V

| Ex. | TT (%) | A (h−1) | P3+4 | | P′ | | C9 | | HC8 | | ROC4 | | C6 | | RR Br (%) |
|---|---|---|---|---|---|---|---|---|---|---|---|---|---|---|---|
| | | | RT (%) | RR (%) | RT (%) | RR (%) | RT (%) | RR (%) | RT (%) | RR (%) | RT (%) | RR (%) | RT (%) | RR (%) | |
| 10a | 25 | 19 | 62,4 | 15,6 | 1,0 | 0,2 | 2,3 | 0,6 | 11,9 | 3,0 | 9,1 | 2,3 | 3,2 | 0,8 | 2,5 |
| 10c | 25,5 | 21 | 66 | 16,8 | 0,9 | 0,2 | 3,5 | 0,9 | 10,9 | 2,8 | 5,2 | 1,3 | 4,1 | 1,0 | 2 |

## Revendications

1. Procédé de préparation d'esters carboxyliques β,γ-insaturés par carbonylation de diènes conjugués par de l'oxyde de carbone, en présence d'un alcool, d'un hydracide halogéné et d'un catalyseur au palladium, ledit procédé étant caractérisé en ce que:
- les produits issus de l'opération de carbonylation sont soumis, en présence d'un chlorure ou d'un bromure d'onium quaternaire choisi parmi les chlorures ou bromures d'ammonium, de phosphonium et d'arsonium quaternaires présentant une température de fusion inférieure à celle à laquelle est réalisée l'opération de carbonylation, à une opération de distillation dans des conditions de température et de pression permettant de récupérer:
une phase gazeuse contenant l'ester d'acide carboxylique β,γ-insaturé correspondant au diène et à l'alcool mis en œuvre, le diène et l'alcool non réagis ainsi que les produits légers volatils, et
une phase liquide homogène constituée d'un mélange du chlorure ou bromure d'onium quaternaire et du catalyseur au palladium ainsi que des produits lourds non volatils;
- après élimination éventuelle des produits lourds, ledit mélange de chlorure ou bromure d'onium quaternaire et de catalyseur au palladium est recyclé sous la forme d'un liquide homogène dans le milieu soumis à l'opération de carbonylation.

2. Procédé selon la revendication 1, caractérisé en ce que le chlorure ou bromure d'onium quaternaire est l'un des sels suivants: chlorure de tétrabutylphosphonium, chlorures de méthyl tri(-octyl, -nonyl, ou -décyl)ammonium ou leurs mélanges, chlorure de méthyltributylammonium, chlorure de benzylbutyldiméthylammonium, ou chlorure de benzylhexadécyldiméthylammonium, chlorure de benzyldiméthyltétradécylammonium, bromure d'hexaldécyltributylphosphonium, bromure de tétrabutylammonium, bromure de tétradodécylammonium, bromure de tétraheptylammonium, bromure de tétrahexylammonium, bromure de tétradécylammonium, bromure de tétraoctadécylammonium, bromure de tétraoctylammonium, bromure de tétrapentylammonium, bromure de tributylheptylammonium, bromure de tributylpentylammonium.

3. Procédé selon la revendication 1 ou 2, caractérisé en ce que la quantité de chlorure ou bromure d'onium quaternaire présente pour réaliser l'opération de distillation correspond à un rapport molaire cation onium quaternaire/palladium d'au moins 2.

4. Procédé selon la revendication 3, caractéærisé en ce que le rapport molaire cation onium quaternaire/palladium est compris entre 5 et 250.

5. Procédé selon l'une quelconque des revendications précédentes, caractérisé en ce que le chlorure ou bromure d'onium quaternaire présent pendant l'opération de distillation est mis en œuvre dans le milieu issu de l'opération de carbonylation en totalité après la première opération de carbonylation ou par fraction au cours d'une série d'opérations de carbonylation-distillation.

6. Procédé selon l'une quelconque des revendications 1 à 4, caractérisé en ce que le chlorure ou bromure d'onium quaternaire présent pendant l'opération de distillation est mis en œuvre en tout ou partie dans le milieu soumis à l'opération de carbonylation.

7. Premier mode d'élimination des produits lourds non volatils, selon le procédé faisant l'objet de l'une quelconque des revendications précédentes, caractérisé en ce que:
- on met en contact la phase liquide homogène constituée du mélange de chlorure ou bromure d'onium quaternaire et de catalyseur au palladium et des produits lourds non volatils, avec un solvant apolaire hydrocarboné aliphatique ou cycloaliphatique, un solvant apolaire non miscible dans ledit solvant polaire et éventuellement une quantité supplémentaire de chlorure ou bromure d'onium quaternaire; et
- après décantation de la phase polaire et de la phase apolaire formées lors de l'opération de mise en contact, on sépare la phase apolaire contenant les produits lourds à récupérer, la phase polaire contenant le mélange de chlorure ou bromure d'onium quaternaire et de catalyseur au palladium et on recycle vers le milieu soumis à l'opération de carbonylation, le mélange de chlorure ou bromure d'onium quaternaire et de catalyseur au palladium soit en solution alcoolique, soit à l'état fondu après élimination du solvant polaire; l'opération de mise en contact étant réalisée avec:
- une quantité de chlorure ou bromure d'onium quaternaire correspondant à un rapport molaire cation onium quaternaire/palladium d'au moins 20;
- des quantités de solvant polaire et de solvant hydrocarboné apolaire au moins égales à celles nécessaires à la décantation du milieu de mise en contact en une phase polaire et une phase apolaire.

8. Premier mode d'élimination des produits lourds selon la revendication 7, caractérisé en ce que l'opération de mise en contact est réalisée en présence:
- d'une quantité de solvant polaire correspondant à un rapport pondéral chlorure ou bromure d'onium quaternaire/solvant polaire d'au moins 0,1;
- d'une quantité de solvant hydrocarboné apolaire correspondant à un rapport pondéral solvant hydrocarboné apolaire/solvant polaire supérieur à 1.

9. Premier mode d'élimination des produits lourds selon la revendication 7 ou 8, caractérisé en ce que l'opération de mise en contact est réalisée en présence:
- d'une quantité de sel d'onium quaternaire correspondant à un rapport molaire cation onium/palladium compris entre 20 et 300;
- d'une quantité de solvant polaire correspondant à un rapport pondéral sel d'onium quaternaire/solvant polaire compris entre 0,25 et 30;
- d'une quantité de solvant apolaire correspondant à un rapport pondéral solvant apolaire/ - solvant polaire supérieur à 2.

10. Premier mode d'élimination des produits lourds selon l'une quelconque des revendications 7 à 9, caractérisé en ce que le solvant polaire présente une constante diélectrique supérieure à 20.

11. Premier mode d'élimination des produits lourds selon la revendication 10, caractérisé en ce que le solvant polaire présente une constante diélectrique supérieure à 30.

12. Premier mode d'élimination des produits lourds, caractérisé en ce que le solvant polaire est l'un des solvants suivants: l'eau, l'acétonitrile, le N,N-diméthylformamide, le diméthylsulfoxyde, le nitrométhane, la N-méthylpyrrolidone.

13. Premier mode d'élimination des produits lourds selon l'une quelconque des revendications 7 à 9, caractérisé en ce que le solvant polaire est un monoalcool aliphatique linéaire ou ramifié contenant de 1 à 4 atomes de carbone.

14. Premier mode d'élimination des produits lourds selon la revendication 13, caractérisé en ce que le solvant polaire est un monoalcool aliphatique linéaire ou ramifié contenant de 1 à 3 atomes de carbone.

15. Premier mode d'élimination des produits lourds selon l'une quelconque des revendications 7 à 14, caractérisé en ce que le solvant apolaire présente une constante diélectrique inférieure à 2,3.

16. Premier mode d'élimination des produits lourds selon la revendication 15, caractérisé en ce que le solvant apolaire présente une constante diélectrique inférieure à 2,1.

17. Premier mode d'élimination des produits lourds selon la revendication 16, caractérisé en ce que le solvant apolaire est un des solvants suivants: le pentane, l'isopentane, l'hexane, le cyclohexane, l'octane, le cyclooctane, le triméthyl-2-2,4 pentane, le décane, le dodécane, le tétradécane, l'hexadécane ou leurs mélanges du type éther de pétrole.

18. Deuxième mode d'élimination des produits lourds non volatils selon le procédé faisant l'objet de l'une quelconque des revendications 1 à 6, caractérisé en ce que:
— on met en contact les constituants de la phase liquide homogène séparée lors de l'opération de distillation avec un solvant apolaire hydrocarboné aliphatique ou cycloaliphatique, à une température inférieure ou égale à la température de prise en masse de la phase liquide homogène;
— on soumet le système solide-liquide obtenu à une opération de filtration;
— on élimine la solution de produits lourds non volatils dans ledit solvant apolaire;
— on transforme le résidu de filtration constitué d'un mélange de chlorure ou bromure d'onium quaternaire et de catalyseur au palladium en un liquide homogène par fusion ou par mise en solution dans un alcool, et
— on recycle ledit liquide homogène obtenu dans le milieu soumis à l'opération de carbonylation.

19. Deuxième mode d'élimination des produits lourds selon la revendication 18, caractérisé en ce que le solvant apolaire présente une constante diélectrique inférieure à 2,3.

20. Deuxième mode d'élimination des produits lourds selon la revendication 19, caractérisé en ce que le solvant apolaire présente une constante diélectrique inférieure à 2,1.

21. Deuxième mode d'élimination des produits lourds selon la revendication 20, caractérisé en ce que le solvant apolaire est un des solvants suivants: le pentane, l'isopentane, l'hexane, le cyclohexane, l'octane, le cyclooctane, le triméthyl-2-2,4 pentane, le décane, le dodécane, le tétradécane, l'hexadécane ou leurs mélanges du type éther de pétrole.

22. Deuxième mode d'élimination des produits lourds selon l'une quelconque des revendications 18 à 21, caractérisé en ce que l'alcool mis en œuvre est un monoalcool contenant de 1 à 4 atomes de carbone.

23. Deuxième mode d'élimination des produits lourds selon la revendication 22, caractérisé en ce que l'alcool mis en œuvre est un monoalcool contenant de 1 à 3 atomes de carbone.

## Patentansprüche

1. Verfahren zur Herstellung von Estern $\beta,\gamma$-ungesättigter Carbonsäuren durch Carbonylieren von konjugierten Dienen mit Kohlenoxid in Gegenwart eines Alkohols, einer Halogenwasserstoffsäure und eines Palladiumkatalysators, dadurch gekennzeichnet, dass
— die bei der Carbonylierung erhaltenen Produkte in Gegenwart eines quaternären Oniumchlorids oder -bromids, ausgewählt unter den quaternären Ammoniumchloriden oder -bromiden, Phosphoniumchloriden oder -bromiden und Arsoniumchloriden oder -bromiden, die eine Schmelztemperatur unterhalb derjenigen aufweisen, bei der die Carbonylierung durchgeführt wird, einer Destillation unterworfen werden unter Bedingungen der Temperatur und des Druckes, die gestatten zurückzugewinnen:
eine Gasphase, die den Ester der dem Dien entsprechenden $\beta,\gamma$-ungesättigten Carbonsäure und den eingesetzten Alkohol, das nicht umgesetzte Dien und den nicht umgesetzten Alkohol sowie die leicht flüchtigen Produkte enthält,
eine homogene flüssige Phase, betehend aus einem Gemisch des quaternären Oniumchlorids oder -bromids und dem Palladiumkatalysator sowie den nicht flüchtigen schweren Produkten,
— nach gegebenenfalls Abtrennung der schweren Produkte das Gemisch aus quaternärem Oniumchlorid oder -bromid und Palladiumkatalysator in Form einer homogenen Flüssigkeit in das der Carbonylierung unterworfene Medium zurückgeführt wird.

2. Verfahren nach Anspruch 1, dadurch gekennzeichnet, dass das quaternäre Oniumchlorid oder -bromid eines der folgenden Salze ist: Tetrabutylphosphoniumchlorid, Methyltrioctylammoniumchlorid, Methyltrinonylammoniumchlorid, Methyltridecylammoniumchlorid oder deren Gemische, Methyltributylammoniumchlorid, Benzylbutyldimethylammoniumchlorid oder Benzylhexadecyldimethylammoniumchlorid, Benzyldimethyltetradecylammoniumchlorid, Hexadecyltributylphosphoniumbromid, Tetrabutylammoniumbromid, Tetradodecylammoniumbromid, Tetraheptylammoniumbromid, Tetrahexylammoniumbromid, Tetradecylammoniumbromid, Tetraoctadecylammoniumbromid, Tetraoctylammoniumbromid, Tetrapentylammoniumbromid, Tributylheptylammoniumbromid, Tributylpentylammoniumbromid.

3. Verfahren nach Anspruch 1 oder 2, dadurch gekennzeichnet, dass die zur Durchführung der Destillation vorhandene Menge quaternäres Oniumchlorid oder -bromid einem Molverhältnis von quaternärem Oniumkation zu Palladium von mindestens 2 entspricht.

4. Verfahren nach Anspruch 3, dadurch gekennzeichnet, dass das Molverhältnis von quaternärem Oniumkation zu Palladium 5 bis 250 beträgt.

5. Verfahren nach einem der vorangehenden Ansprüche, dadurch gekennzeichnet, dass das während der Destillation vorhandene quaternäre Oniumchlorid oder -bromid in das bei der Carbonylierung erhaltenen Medium insgesamt nach der ersten Carbonylierung oder anteilweise im Verlauf einer Reihe von Carbonylierungs-Destillationen eingesetzt wird.

6. Verfahren nach einem der Ansprüche 1 bis 4, dadurch gekennzeichnet, dass das während der Destillation vorhandene quaternäre Oniumchlorid oder -bromid insgesamt oder teilweise in das Medium eingesetzt wird, das der Carbonylierung unterworfen wird.

7. Erste Ausführungsform zur Abtrennung der nicht flüchtigen schweren Produkte bei dem Verfahren nach einem der vorangehenden Ansprüche, dadurch gekennzeichnet, dass

— man die homogene flüssige Phase, die aus dem Gemisch des quaternären Oniumchlorids oder -bromids und dem Palladiumkatalysator sowie den nicht flüchtigen schweren Produkten besteht, mit einem aliphatischen oder cycloaliphatischen apolaren Kohlenwasserstofflösungsmittel, einem mit diesem apolaren Lösungsmittel nicht mischbaren polaren Lösungsmittel und gegebenenfalls einer zusätzlichen Menge quaternärem Oniumchlorid oder -bromid in Berührung bringt, und

— nach dem Absitzenlassen der bei diesem Inberührungmiteinanderbringen entstandenen polaren und apolaren Phase die apolare Phase, die die schweren Produkte enthält, die zurückgewonnen werden sollen, und die polare Phase, die das Gemisch aus quaternärem Oniumchlorid oder -bromid und Palladiumkatalysator enthält, trennt und in das der Carbonylierung unterworfene Medium das Gemisch aus quaternärem Oniumchlorid oder -bromid und Palladiumkatalysator zurückführt, entweder in alkoholischer Lösung oder in geschmolzenem Zustande nach Abtrennen des polaren Lösungsmittels, wobei das Inberührungmiteinanderbringen durchgeführt wird mit
— einer Menge quaternäres Oniumchlorid oder -bromid, die einem Molverhältnis von quaternärem Oniumkation zu Palladium von mindestens 20 entspricht,
— Mengen an polarem Lösungsmittel und apolarem Kohlenwasserstofflösungsmittel, die mindestens gleich sind denjenigen, die beim Absitzenlassen des Mediums des Inberührungbringens in eine polare Phase und in eine apolare Phase notwendig sind.

8. Erste Ausführungsform der Abtrennung von schweren Produkten nach Anspruch 7, dadurch gekennzeichnet, dass man das Inberührungbringen durchführt in Gegenwart von:
— einer Menge polaren Lösungsmittels, die einem Gewichtsverhältnis quaternäres Oniumchlorid oder -bromid zu polarem Lösungsmittel von mindestens 0,1 entspricht,
— einer Menge apolaren Kohlenwasserstofflösungsmittels, die einem Gewichtsverhältnis apolares Kohlenwasserstofflösungsmittel zu polarem Lösungsmittel von über 1 entspricht.

9. Erste Ausführungsform der Abtrennung der schweren Produkte nach Anspruch 7 oder 8, dadurch gekennzeichnet, dass man das Inberührrungbringen durchführt in Gegenwart von:
— einer Menge quaternären Oniumsalzes, die einem Molverhältnis von Oniumkation zu Palladium von 20 bis 300 entspricht,
— einer Menge polaren Lösungsmittels, die einem Gewichtsverhältnis quaternäres Oniumsalz zu polarem Lösungsmittel von 0,25 bis 30 entspricht,
— einer Menge apolaren Lösungsmittels, die einem Gewichtsverhältnis apolares Lösungsmittel zu polarem Lösungsmittel von mehr als 2 entspricht.

10. Erste Ausführungsform der Abtrennung von schweren Produkten nach einem der Ansprüche 7 bis 9, dadurch gekennzeichnet, dass das polare Lösungsmittel eine Dielektrizitätskonstante von über 20 aufweist.

11. Erste Ausführungsform der Abtrennung von schweren Produkten nach Anspruch 10, dadurch gekennzeichnet, dass das polare Lösungsmittel eine Dielektrizitätskonstante von über 30 aufweist.

12. Erste Ausführungsform der Abtrennung von schweren Produkten, dadurch gekennzeichnet, dass das polare Lösungsmittel eines der folgenden Lösungsmittel ist: Wasser, Acetonitril, N,N-Dimethylformamid, Dimethylsulfoxid, Nitromethan, N-Methylpyrrolidon.

13. Erste Ausführungsform der Abtrennung von schweren Produkten nach einem der Ansprüche 7 bis 9, dadurch gekennzeichnet, dass das polare Lösungsmittel ein linearer oder verzweigter aliphatischer Monoalkohol mit 1 bis 4 Kohlenstoffatomen ist.

14. Erste Ausführungsform der Abtrennung von schweren Produkten nach Anspruch 13, dadurch gekennzeichnet, dass das polare Lösungsmittel ein linear oder verzweigter aliphatischer Monoalkohol mit 1 bis 3 Kohlenstoffatomen ist.

15. Erste Ausführungsform der Abtrennung von schweren Produkten nach einem der Ansprüche 7 bis 14, dadurch gekennzeichnet, dass das apolare Lösungsmittel eine Dielektrizitätskonstante unter 2,3 aufweist.

16. Erste Ausführungsform der Abtrennung der schweren Produkte nach Anspruch 15, dadurch gekennzeichnet, dass das apolare Lösungsmittel eine Dielektrizitätskonstante unter 2,1 aufweist.

17. Erste Ausführungsform der Abtrennung von schweren Produkten nach Anspruch 16, dadurch gekennzeichnet, dass das apolare Lösungsmittel eines der folgenden Lösungsmittel ist: Pentan, Isopentan, Hexan, Cyclohexan, Octan, Cyclooctan, 2,2,4-Trimethylpentan, Decan, Dodecan, Tetradecan, Hexadecan oder deren Gemische vom Typ Petrolether.

18. Zweite Ausführungsform der Abtrennung von nicht flüchtigen schweren Produkten bei dem Verfahren nach einem der Ansprüche 1 bis 6, dadurch gekennzeichnet, dass
— man die Bestandteile der bei der Destillation abgetrennten homogenen flüssigen Phase in Berührung bringt mit einem apolaren aliphatischen oder cycloaliphatischen Kohlenwasserstofflösungsmittel bei einer Temperatur unter oder gleich der Temperatur, bei der die flüssige homogene Phase fest wird,
— das erhaltene fest-flüssig-System filtriert,
— die Lösung der nicht flüchtigen schweren Produkte in dem apolaren Lösungsmittel abtrennt,
— den Filtrationsrückstand, bestehend aus einem Gemisch aus quaternärem Oniumchlorid oder -bromid und Palladiumkatalysator, durch Schmelzen oder durch Auflösen in einem Alkohol in eine homogene Flüssigkeit überführt,
— diese erhaltene homogene Flüssigkeit in das Medium zurückführt, das der Carbonylierung unterworfen wird.

19. Zweite Ausführungsform der Abtrennung der schweren Produkte nach Anspruch 18, dadurch gekennzeichnet, dass das apolare Lösungsmittel eine Dielektrizitätskonstante unter 2,3 aufweist.

20. Zweite Ausführungsform der Abtrennung der schweren Produkte nach Anspruch 19, dadurch gekennzeichnet, dass das apolare Lösungsmittel eine Dielektrizitätskonstante unter 2,1 aufweist.

21. Zweite Ausführungsform der Abtrennung der schweren Produkte nach Anspruch 20, dadurch gekennzeichnet, dass das apolare Lösungsmittel eines der folgenden Lösungsmittel ist: Pentan, Isopentan, Hexan, Cyclohexan, Octan, Cyclooctan, 2,2,4-Trimethylpentan, Decan, Dodecan, Tetradecan, Hexadecan oder deren Gemische vom Typ Petrolether.

22. Zweite Ausführungsform der Abtrennung von schweren Produkten nach einem der Ansprüche 18 bis 21, dadurch gekennzeichnet, dass der eingesetzte Alkohol ein Monoalkohol mit 1 bis 4 Kohlenstoffatomen ist.

23. Zweite Ausführungsform der Abtrennung von schweren Produkten nach Anspruch 22, dadurch gekennzeichnet, dass der eingesetzte Alkohol ein Monoalkohol mit 1 bis 3 Kohlenstoffatomen ist.

## Claims

1. Process for the preparation of esters of $\beta,\gamma$-unsaturated carboxylic acids by the carbonylation of conjugated dienes with carbon monoxide, in the presence of an alcohol, a halogenated hydracid and a palladium catalyst, the said process being characterised in that:
— the products resulting from the carbonylation operation are subjected, in the presence of a quaternary onium chloride or bromide chosen from amongst quaternary ammonium, phosphonium and arsonium chlorides or bromides having a melting point below that at which the carbonylation operation is carried out, to a distillation operation under temperature and pressure conditions which make it possible to recover:
a gas phase containing the ester of the $\beta,\gamma$-unsaturated carboxylic acid corresponding to the diene and the alcohol used, the unreacted diene and alcohol and also the volatile products of low molecular weight; and
a homogeneous liquid phase consisting of a mixture of the quaternary onium chloride or bromide and the palladium catalyst, and also of the non-volatile products of high molecular weight; and
— after removal of the products of high molecular weight, if appropriate, the said mixture of quaternary onium chloride or bromide and palladium catalyst is recycled, in the form of a homogeneous liquid, into the medium subjected to the carbonylation operation.

2. Process according to Claim 1, characterised in that the quaternary onium chloride or bromide is one of the following salts: tetrabutylphosphonium chloride, methyl-tri-(octyl, nonyl or decyl)-ammonium chlorides or their mixtures, methyltributylammonium chloride, benzylbutyldimethylammonium chloride, benzylhexadecyldimethylammonium chloride, benzyldimethyltetradecylammonium chloride, hexadecyltributylphosphonium bromide, tetrabutylammonium bromide, tetradodecylammonium bromide, tetraheptylammonium bromide, tetrahexylammonium bromide, tetradecylammonium bromide, tetraoctadecylammonium bromide, tetraoctylammonium bromide, tetrapentylammonium bromide, tributylheltylammonium bromide and tributylpentylammonium bromide.

3. Process according to Claims 1 or 2, characterised in that the amount of quaternary onium chloride or bromide present for carrying out the distillation operation corresponds to a molar ratio quaternary onium cation/palladium of at least 2.

4. Process according to Claim 3, characterised in that the molar ratio quaternary onium cation/palladium is between 5 and 250.

5. Process according to any one of the preceding claims, characterised in that the quaternary onium chloride or bromide present during the distillation operation is introduced into the medium resulting from the carbonylation operation, either in total after the first carbonylation operation, or in portions during a series of carbonylation/distillation operations.

6. Process according to any one of Claims 1 to 4, characterised in that all or part of the quaternary onium chloride or bromide present during the distillation operation is used in the medium subjected to the carbonylation operation.

7. First method of removing the non-volatile products of high molecular weight, in accordance with the process forming the subject of any one of the preceding claims, characterised in that:

the homogeneous liquid phase, consisting of the mixture of quaternary onium chloride or bromide and palladium catalyst, and of the non-volatile products of high molecular weight, is brought into contact with an apolar aliphatic or cycloaliphatic hydrocarbon solvent, a polar solvent which is immiscible with the said apolar solvent and, if appropriate, an additional amount of quaternary onium chloride or bromide; and

after decantation of the polar phase and the apolar phase formed during the contacting operation, the apolar phase containing the products of high molecular weight to be recovered is separated from the polar phase containing the mixture of quaternary onium chloride or bromide and palladium catalyst, and the mixture of quaternary onium chloride or bromide and palladium catalyst, either in the form of an alcohol solution, or in the molten state after removal of the polar solvent, is recycled into the medium subjected to the carbonylation operation, the contacting operation being carried out with:

an amount of quaternary onium chloride or bromide corresponding to a molar ratio quaternary onium cation/palladium of at least 20; and

amounts of polar solvent and apolar hydrocarbon solvent which are at least equal to those required for the decantation of the contacting medium to form a polar phase and an apolar phase.

8. First method of removing the products of high molecular weight, according to Claim 7, characterised in that the contacting operation is carried out in the presence of:

an amount of polar solvent corresponding to a weight ratio quaternary onium chloride or bromide/polar solvent of at least 0.1; and

an amount of apolar hydrocarbon solvent corresponding to a weight ratio apolar hydrocarbon solvent/polar solvent of more than 1.

9. First method of removing the products of high molecular weight, according to Claims 7 or 8, characterised in that the contacting operation is carried out in the presence of:

an amount of quaternary onium salt corresponding to a molar ratio onium cation/palladium of between 20 and 300;

an amount of polar solvent corresponding to a weight ratio quaternary onium salt/polar solvent of between 0.25 and 30; and

an amount of apolar solvent corresponding to a weight ratio apolar solvent/polar solvent of more than 2.

10. First method of removing the products of high molecular weight, according to any one of Claims 7 to 9, characterised in that the polar solvent has a dielectric constant of more than 20.

11. First method of removing the products of high molecular weight, according to Claim 10, characterised in that the polar solvent has a dielectric constant of more than 30.

12. First method of removing the products of high molecular weight, characterised in that the polar solvent is one of the following solvents: water, acetonitrile, N,N-dimethylformamide, dimethyl sulphoxide, nitromethane and N-methylpyrrolidone.

13. First method of removing the products of high molecular weight, according to any one of Claims 7 to 9, characterised in that the polar solvent is a linear or branched aliphatic monoalcohol containing from 1 to 4 carbon atoms.

14. First method of removing the products of high molecular weight, according to Claim 13, characterised in that the polar solvent is a linear or branched aliphatic monoalcohol containing from 1 to 3 carbon atoms.

15. First method of removing the products of high molecular weight, according to any one of Claims 7 to 14, characterised in that the apolar solvent has a dielectric constant of less than 2.3.

16. First method of removing the products of high molecular weight, according to Claim 15, characterised in that the apolar solvent has a dielectric constant of less than 2.1.

17. First method of removing the products of high molecular weight, according to Claim 16, characterised in that the apolar solvent is one of the following solvents: pentane, isopentane, hexane, cyclohexane, octane, cyclo-octane, 2,2,4-trimethylpentane, decane, dodecane, tetradecane, hexadecane or their mixtures of the petroleum ether type.

18. Second method of removing the non-volatile products of high molecular weight, in accordance with the process forming the subject of any one of Claims 1 to 6, characterised in that:

the constituents of the homogeneous liquid phase, separated off during the distillation operation, are brought into contact with an apolar aliphatic or cycloaliphatic hydrocarbon solvent at a temperature which is below or equal to the solidification temperature of the homogeneous liquid phase;

the solid-liquid system obtained is subjected to a filtration operation;

the solution of non-volatile products of high molecular weight in the said apolar solvent is removed;

the filtration residue, consisting of a mixture of quaternary onium chloride or bromide and palladium catalyst, is converted to a homogeneous liquid by melting or by dissolution in an alcohol; and

the said homogeneous liquid obtained is recycled into the medium subjected to the carbonylation operation.

19. Second method of removing the products of high molecular weight, according to Claim 18, characterised in that the apolar solvent has a dielectric constant of less than 2.3.

20. Second method of removing the products of high molecular weight, according to Claim 19, characterised in that the apolar solvent has a dielectric constant of less than 2.1.

21. Second method of removing the products of high molecular weight, according to Claim 20, characterised in that the apolar solvent is one of the following solvents: pentane, isopentane, hexane, cyclohexane, octane, cyclo-octane, 2,2,4-trimethylpentane, decane, dodecane, tetradecane, hexadecane or their mixtures of the petroleum ether type.

22. Second method of removing the products of high molecular weight, according to any one of Claims 18 to 21, characterised in that the alcohol used is a monoalcohol containing from 1 to 4 carbon atoms.

23. Second method of removing the products of high molecular weight, according to Claim 22, characterised in that the alcohol used is a monoalcohol containing from 1 to 3 carbon atoms.